(19) Europäisches Patentamt
European Patent Office
Office européen des brevets

(11) Publication number: **0 065 864 B1**

# (12) EUROPEAN PATENT SPECIFICATION

(45) Date of publication of patent specification: **28.08.85**

(21) Application number: **82302494.8**

(22) Date of filing: **17.05.82**

(51) Int. Cl.[4]: **C 07 D 498/10**, A 61 K 31/535 // (C07D498/10, 265:00, 221:00)

(54) **Anti-hypertensive 1-substituted spiro (piperidine-oxobenzoxazines).**

(30) Priority: **18.05.81 US 264779**

(43) Date of publication of application:
**01.12.82 Bulletin 82/48**

(45) Publication of the grant of the patent:
**28.08.85 Bulletin 85/35**

(84) Designated Contracting States:
**AT BE CH DE FR GB IT LI LU NL SE**

(56) References cited:
**GB-A-1 292 787**

(73) Proprietor: **SYNTEX (U.S.A.) INC.**
**3401 Hillview Avenue**
**Palo Alto, California 94304 (US)**

(72) Inventor: **Roszkowski, Adolph Peter**
**15060 Sobey Road**
**Saratoga California 95070 (US)**
Inventor: **Clark, Robin Douglas**
**2295 Columbia Street**
**Palo Alto California 94306 (US)**
Inventor: **Kluge, Arthur Frederick**
**1683 Parkhills Avenue**
**Los Altos California 94022 (US)**

(74) Representative: **Armitage, Ian Michael et al**
**MEWBURN ELLIS & CO. 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

High blood pressure is a widespread condition which has become, especially in recent years, the object of intensive concern and effort with respect to alleviating the life-threatening effects of its presence. The invention herein concerns a class of 1-substituted spiropiperidine heterocycles which are effective in the central nervous system (CNS) and are useful in treating hypertension.

Background of the Invention

It has become clear that at least some of the factors controlling blood pressure reside in the CNS. By altering the state of the receptors for adrenergic compounds through providing compounds suitable for binding at these sites, outcomes such as vasolidation or constriction, heartbeat rate acceleration or slowdown, and heart blood vessel pressure changes can be effected.

The compounds of the present invention have been shown to lower blood pressure. These compounds are spiro[piperidine-oxobenzooxazines]. The literature describes certain other compounds with superificially similar structures, different from the compounds of this invention. For example, U.S. 4,224,333, issued September 30, 1980 discloses 2-(benzodioxan-2-yl)-2-hydroxyethylpiperidines; substituted with 2H-indol-2-ones at the 4-position of the piperidine ring, as antihypertensives.

The invention herein concerns novel compounds of the formula:

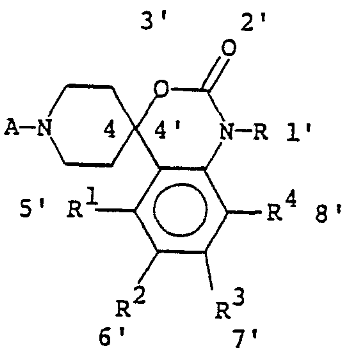

(I)

and the pharmaceutically acceptable acid addition salts thereof, wherein

$R^1$, $R^2$, $R^3$ and $R^4$ are each independently hydrogen, hydroxy, lower alkyl, lower alkoxy or halo;

R is hydrogen or lower alkyl; and

A is selected from:

2-(benzodioxan-2-yl)-2-hydroxyethyl;

ω-(benzodioxan-2-yl)-alkyl (where alkyl is a straight or branched chain hydrocarbon of 1—4 carbons);

3-(aryloxy)-2-hydroxypropyl, wherein aryloxy is phenyloxy optionally substituted by 1, 2 or 3 moieties selected from lower alkyl, lower alkoxy, halo, lower alkylsulfamido, lower alkoxycarbonyl, cyano and trifluoromethyl;

ω-arylalkyl wherein alkyl is as hereinabove defined and wherein aryl is phenyl optionally substituted by 1, 2 or 3 moieties selected from lower alkyl, lower alkoxy, halo, lower alkylsulfamido, lower alkoxycarbonyl, cyano and trifluoromethyl;

ω-aryl-ω-oxoalkyl, wherein aryl and alkyl are as hereinabove defined;

ω-aryl-ω-hydroxyalkyl, wherein alkyl and aryl are as herein defined; and

ω-aryloxyalkyl, wherein alkyl and aryloxy are as herein defined.

The compounds of Formula I are antihypertensives. Therefore, in two other aspects, the invention concerns a compound of Formula I or a pharmaceutically acceptable salt thereof for use in mammals; and pharmaceutical compositions containing a compound of Formula I or a pharmaceutically acceptable salt thereof.

In a fourth aspect, the invention concerns methods of preparing the compounds of Formula I.

As used herein:

"Pharmaceutically acceptable acid addition salt" refers to those salts which retain the biological effectiveness and properties of the free bases and which are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, menthanesulfonic acid, ethanesulfonic acid, p-toluenesulfonic acid or salicylic acid.

"Alkyl" and "lower alkyl" mean a branched or unbranched saturated hydrocarbon chain of 1, 2, 3, or 4 carbons, such as, for example, methyl, ethyl, n-propyl, i-propyl, n-butyl, i-butyl or t-butyl.

"Lower alkoxy" means the substituent —OR wherein R is lower alkyl as defined herein.

"Halo" refers to fluoro, chloro or bromo.

"Optional" or "optionally" means that the subsequently described event or circumstance may or may not occur, and that the description includes instances where said event or circumstance occurs, and instances in which it does not. For example, "optionally substituted phenyl" means that the phenyl may or may not be substituted and that the description includes both unsubstituted phenyl and phenyl wherein there is substitution.

"Substituted phenyl" as used herein means that one or more hydrogens, preferably 1, 2 or 3 hydrogens, of the phenyl ring are replaced by moieties selected from lower alkyl, lower alkoxy, halo, lower alkylsulfamido, lower alkoxycarbonyl, cyano, and trifluoromethyl. In the context of the present invention, said replacement may be at any position of the phenyl ring.

The moieties represented by A in formula I are as follows:

2-(benzodioxan-2-yl)-2-hydroxyethyl represents

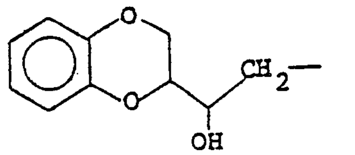 ;

ω-(benzodioxan-2-yl)-alkyl represents

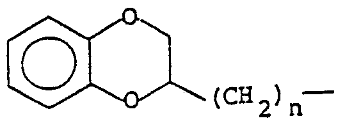 ;

(n = 1-4)

3-(aryloxy)-2-hydroxypropyl represents

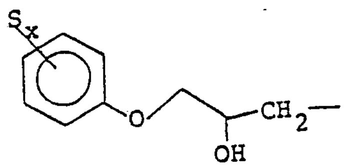

wherein

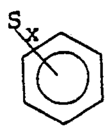

represents phenyl optionally substituted by 1, 2 or 3 moieties selected from lower alkyl, lower alkoxy, halo, lower alkylsulfamido, lower alkoxycarbonyl, cyano and trifluoromethyl, as hereinabove defined;

ω-aryloxyalkyl represents

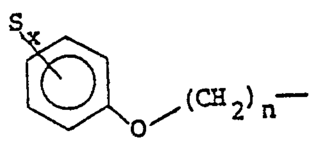

(n = 1-4)

wherein

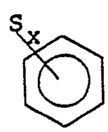

is as herein defined;

ω-aryl-ω-oxoalkyl represents

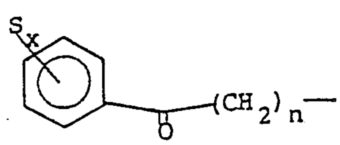

(n = 0-3)

wherein

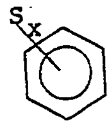

is as herein defined

ω-aryl-ω-hydroxyalkyl represents

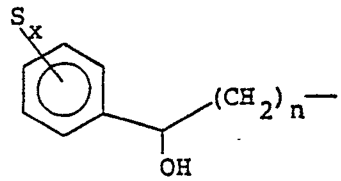

(n = 0-3)

wherein

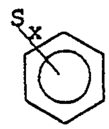

is as herein defined; and

ω-arylalkyl represents

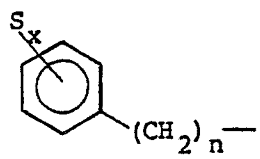

(n = 1-4)

wherein

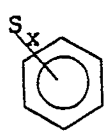

is as herein defined.

Thus, in general, "aryl" represents

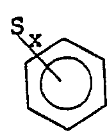

and "aryloxy" represents

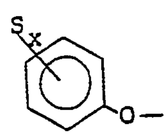

wherein $S_x$ represents substitution as herein defined.

General Methods of Preparation

All of the compounds of the invention are prepared by employing the general approaches shown in Reaction Sequences I, II or III as discussed below.

A compound of the formula:

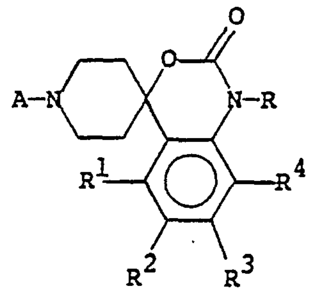

(I)

and the pharmaceutically acceptable acid addition salts thereof, wherein

$R^1$, $R^2$, $R^3$ and $R^4$ are each independently hydrogen, hydroxy, lower alkyl, lower alkoxy or halo;

R is hydrogen or lower alkyl; and

A is selected from:

2-(benzodioxan-2-yl)-2-hydroxyethyl;

ω-(benzodioxan-2-yl)-alkyl (where alkyl is a straight or branched chain hydrocarbon of 1—4 carbons);

3-(aryloxy)-2-hydroxypropyl, wherein aryloxy is phenyloxy optionally substituted by 1, 2 or 3 moieties selected from lower alkyl, lower alkoxy, halo, lower alkylsulfamido, lower alkoxycarbonyl, cyano and trifluoromethyl;

ω-arylalkyl where alkyl is as hereinabove described and wherein aryl is phenyl optionally substituted by 1, 2 or 3 moieties selected from lower alkyl, lower alkoxy, halo, lower alkylsulfamido, lower alkoxycarbonyl, cyano and trifluoromethyl;

ω-aryl-ω-oxoalkyl, wherein alkyl and aryl are as herein defined;

ω-aryl-ω-hydroxyalkyl, wherein alkyl and aryl are as herein defined; and

ω-aryloxyalkyl, wherein alkyl and aryl are as herein defined;

may be prepared by

a) converting a compound of the formula

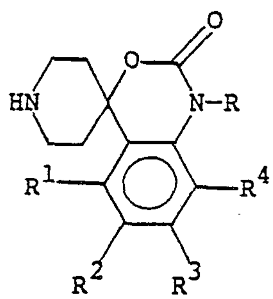

(IV)

wherein $R^1$, $R^2$, $R^3$, and $R^4$, are as herein defined, and R is hydrogen to a compound of formula IV wherein R is lower alkyl where alkyl is as herein defined;

b) treating a compound of formula IV wherein $R^1$, $R^2$, $R^3$, and $R^4$ are as herein defined and R is hydrogen or lower alkyl with a compound selected from the group

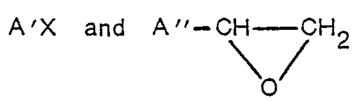

wherein A′ is ω-(benzodioxan-2-yl)alkyl, ω-aryloxyalkyl, ω-arylalkyl, or ω-aryl-ω-oxoalkyl; and A″ is benzodioxan-2-yl or aryloxymethyl wherein alkyl and aryl are as herein defined to form a compound of formula I; or

c) converting a compound of formula I wherein R is hydrogen and $R^1$, $R^2$, $R^3$ and $R^4$ have the above meanings to the corresponding product of formula I wherein R is lower alkyl; or

d) converting a compound of formula I wherein $R^1$, $R^2$, $R^3$ and $R^4$ are as herein defined, R is hydrogen or lower alkyl, and A is ω-aryl-ω-oxyalkyl into the corresponding compound of formula I wherein A is ω-aryl-ω-hydroxyalkyl.

Or this compound (I) may be prepared by

(a) treating a compound of the formula

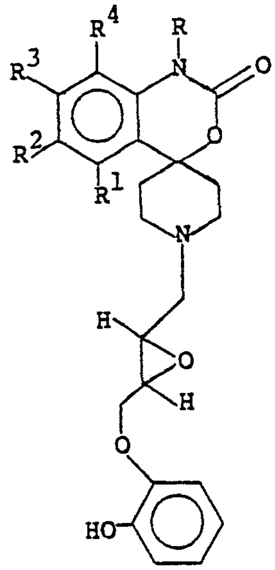

wherein $R^1$, $R^2$, $R^3$, $R^4$, and R are as herein defined with a strong base to form a compound of formula I; or

(b) converting a compound of formula I wherein R is hydrogen to a compound of formula I wherein R is lower alkyl.

Or compound (I) may be prepared by

a) treating a compound of the formula

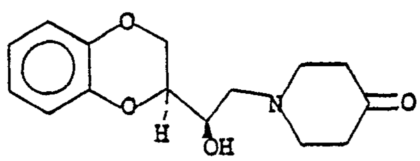 (XIV)

with a compound of the formula

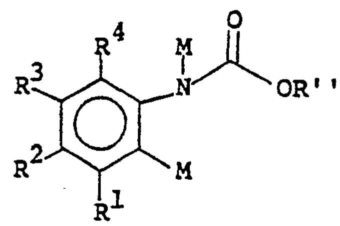

wherein $R^1$, $R^2$, $R^3$ and $R^4$ are as herein defined, M is metal to form a compound of formula I wherein R is hydrogen and $R^1$, $R^2$, $R^3$ and $R^4$ have the above meanings; followed by

b) deprotecting a compound of formula XVII

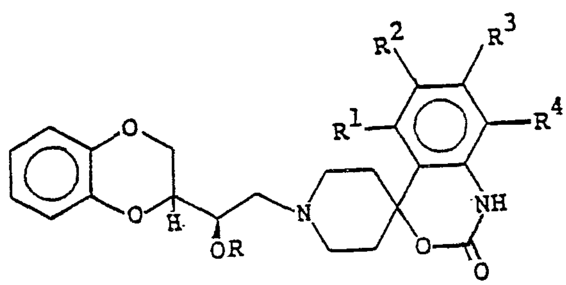

wherein R is an alcohol protecting group and $R^1$, $R^2$, $R^3$, and $R^4$ have the above meanings to obtain a corresponding compound of formula I wherein R is hydrogen, or

c) converting a compound of formula XI

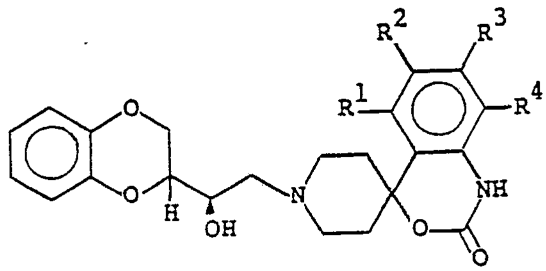

wherein R′, $R^2$, $R^3$ and $R^4$ have the above meanings to a corresponding compound of formula I wherein R is lower alkyl.

In all of these cases

the free base of the compound of formula I may be converted to a pharmaceutically acceptable acid addition salt; or

a salt of the compound of formula I may be converted to a free base; or

a salt of the compound of formula I may be converted to another salt.

Reaction Sequence I

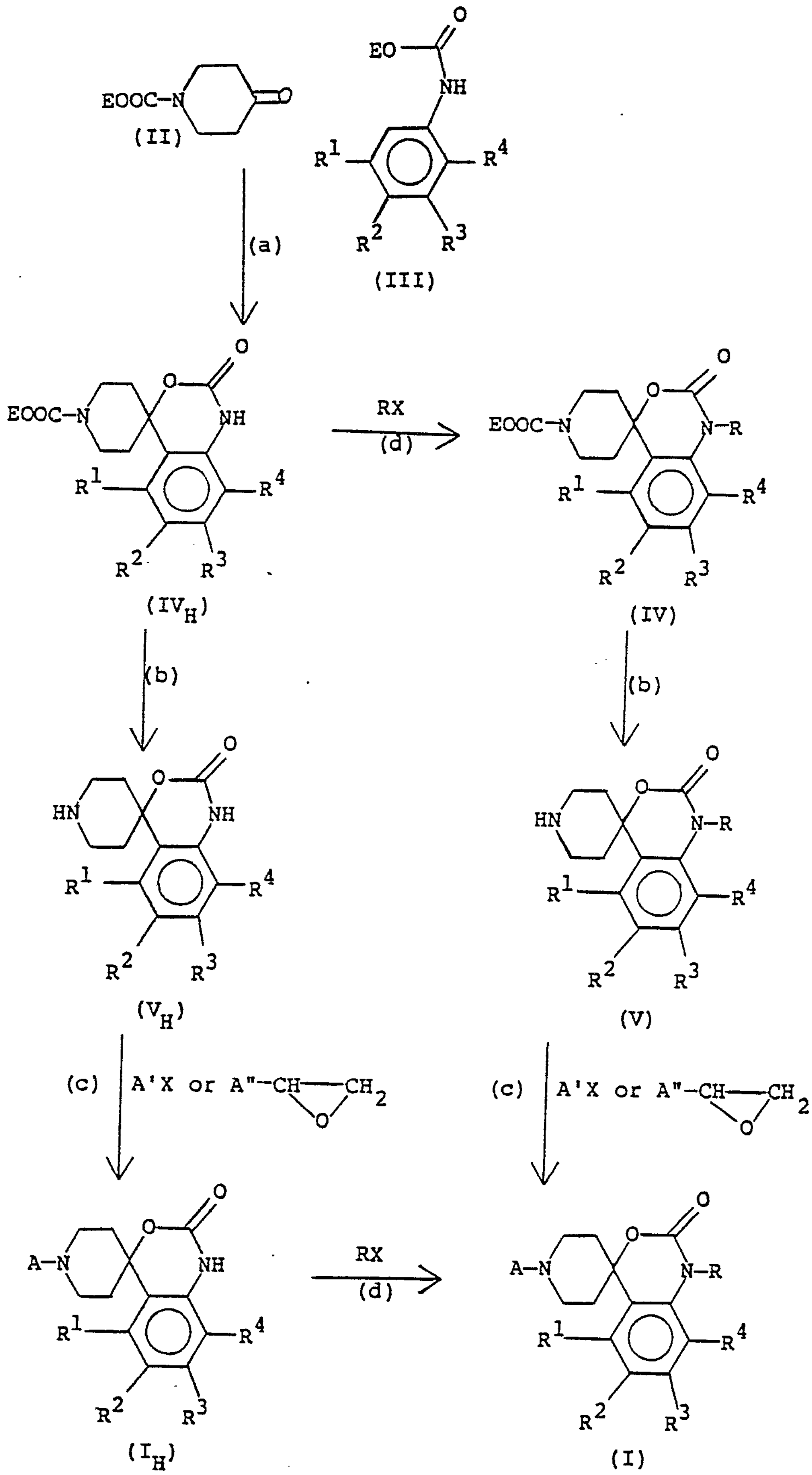


(II) (III) ($IV_H$) (IV) ($V_H$) (V) ($I_H$) (I)

(E represents an alkyl (where alkyl is as herein defined) group in an ester linkage with the carboxyl group shown. t-Butyl is the most preferred among such esterifying alkyl groups. X represents a leaving group such as, for example, a halide or sulfonic acid ester; preferably a halide. The subscript "H" below the number of a formula designates the forms of the compound wherein R is hydrogen).

A' represents the subclass of embodiments of A which is the group consisting of ω-(benzodioxan-2-yl)alkyl, ω-aryloxyalkyl, ω-arylalkyl, and ω-aryl-ω-oxoalkyl;

A" represents a radical selected from benzodioxan-2-yl, and aryloxymethyl.

(An additional step is required to prepare those compounds wherein A is ω-aryl-ω-hydroxyalkyl: by reducing the corresponding ω-aryl-ω-oxoderivative. One method is hydrogenation using a catalyst. One suitable cdatalyst is palladium on carbon).

The starting materials of formulas II and III,

A'X, 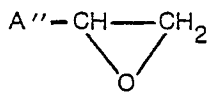 and RX

are commercially available or may be prepared by standard methods. A particularly useful method of preparing the benzodioxanyl ethyl epoxides is described in U.S. Patent 4,212,808.

In carrying out step (a) of Reaction Sequence I, the compound of Formula III, preferably that wherein E represents a tertiary butyl moiety, is first treated with a strong base such as a metal hydride or a metal alkide, preferably tertiary butyl lithium, in the presence of an inert aprotic solvent such as for example, tetrahydrofuran (THF), dimethylformamide (DMF), or dimethylsulfoxide (DMSO); preferably DMF. An excess of the base is used, about 1.5—4 moles of base to 1 mole of the compound of Formula III, preferably 1.5—2 moles of base. The reaction is kept at a temperature of between about 0° to −80°C, preferably between −20° to −80°C for about 0.5—4 hours, preferably 1—3 hours.

The compound of Formula II, preferably that wherein E represents a tertiary butyl ester, is then added dropwise until an approximately stoichiometric amount is added to the reaction mixture as prepared in the previous paragraph, over a period of about 5—60 minutes, preferably about 10 minutes at a temperature of about −20° to −80°C, prefeably about −80°C.

The compound of formula $IV_H$ may be isolated if desired, or the reaction mixture treated directly with a deprotecting agent to remove the alkoxycarbonyl moiety from the nitrogen to give $V_H$. The deprotection is carried out under acid conditions by methods known in the art.

The compounds of either Formula $I_H$ or $IV_2$ may be alkylated by treating with a suitable alkyl halide in a molar excess of about 1.2—5 moles alkyl halide to substrate compound, preferably 1.5—2 molar excess, for about 1—5 hours at about 25—100°C. Formula $I_H$ or $IV_H$ must be preliminarily treated with strong base, such as for example, an alkali metal halide prior to the alkyl halide treaatment step. [Step(s) (d)].

The deprotected form of the compound, formula V, (or $V_H$) is treated with a suitable epoxide or halide in a manner substantially analogous to that described in U.S. Patent 4,224,333. In this procedure, with respect to the epoxide, the substrate spiro[piperidine-4,4'-2'-oxo-3',1'-benzooxazine] and the appropriate epoxide, in approximately equimolar ratio, are dissolved in a solvent of appropriate polarity such as alcohol/hydrocarbon mixtures, for example, methanol/benzene, methanol/toluene, or ethanol/toluene, preferably methanol/toluene. The solution is then cooled to about −10°C to +50°C, preferably +5°C to +10°C and the resulting compound of formula $I_H$ or I is then precipitated. With respect to the halide, the substrate spiro[piperidine-4,4'-2'-oxo-3',1'-benzooxazine] and the appropriate bromide or chloride in approximately equimolar ratio, are dissolved in a non-protic, mildly basic solvent, formed from an aprotic solvent and a tertiary amine such as, for example, dimethylformamide/triethylamine, tetrahydrofuran/triethylamine, or dimethylformamide/pyridine, preferably dimethylformamide/triethylene amine. The mixture is then heated to about 40° to about 100°C, preferably 55° to 65°C for about 2 hours to 10 hours, preferably 4 to 6 hours. The mixture is then quenched with water, and the product extracted into an organic solvent. In either case, the resulting compound of Formula I may be isolated by conventional means.

Reaction Sequence II

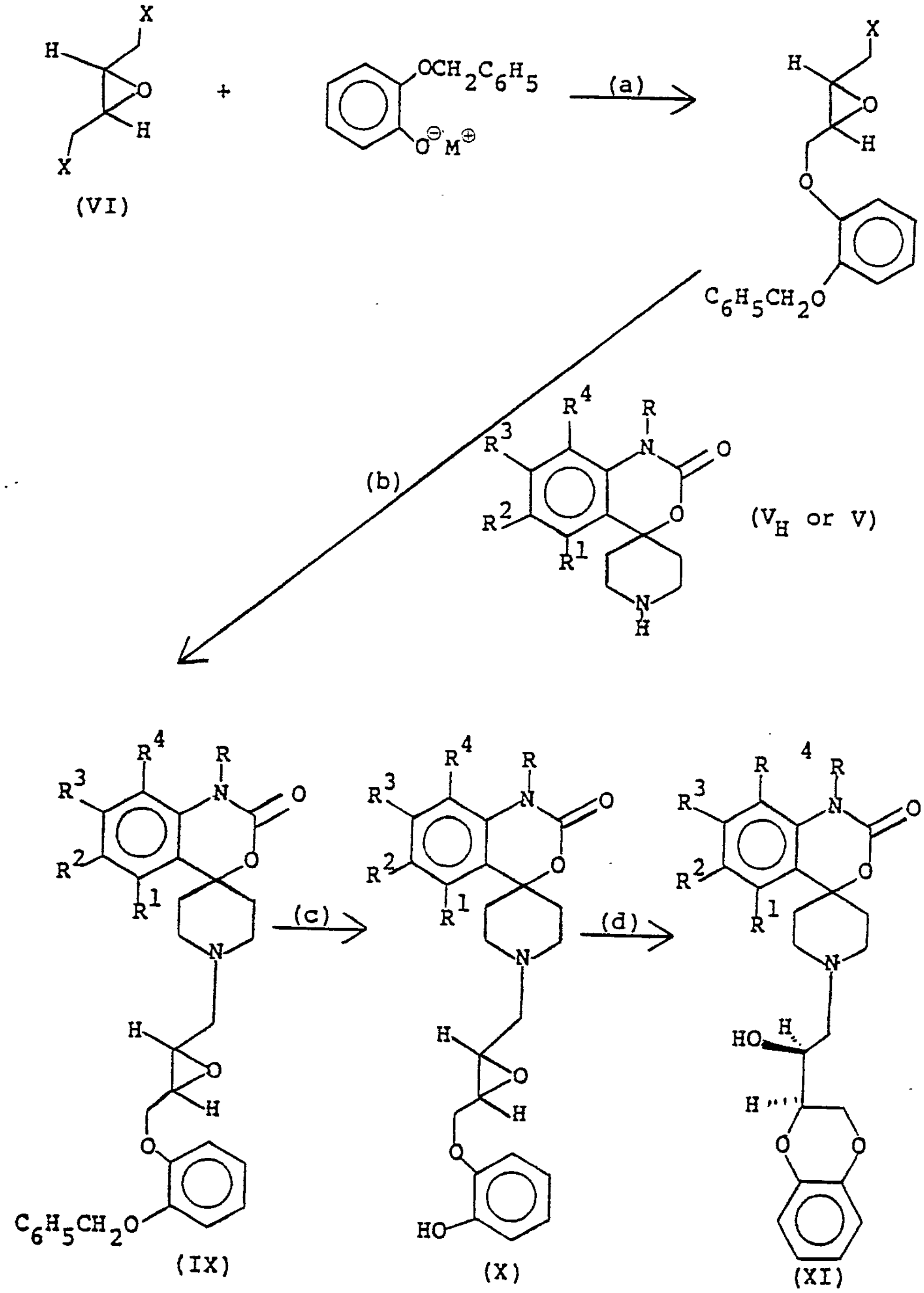


(VI)

($V_H$ or V)

(IX) (X) (XI)

In carrying out step (a) of Reaction Sequence II the compound of formula VI, an epoxide, preferably a trans epoxide, where X is a halogen, preferably chloro or bromo, is reacted in an organic solvent, for example an alcohol wherein alkyl is a straight or branched hydrocarbon chain of one to four carbon atoms, a lower alkyl ketone wherein alkyl is as herein defined, such as for example, acetone, or a lower dialkyl amide wherein alkyl is as herein defined, such as for example dimethylformamide, at a temperature of about 30° to 150°C, preferably at or near reflux and for a time of from about 1 to 24 hours, preferably 1 to 4 hours with a strong base such as a metal aryloxide (formula VII) to afford the compound of formula VIII.

The compound of formula VIII is reacted with a compound of formula V or $V_H$ wherein R is defined as hydrogen or a lower alkyl group wherein alkyl is defined as a straight or branched hydrocarbon chain of one to four carbon atoms, and $R^1$, $R^2$, $R^3$ and $R^4$ are independently hydrogen, hydroxy, lower alkyl or lower alkoxy, in an organic solvent, for example a lower dialkylamide wherein alkyl is as herein defined such as dimethylformamide, a lower alkyl nitrile wherein alkyl is as herein defined, such as acetonitrile, or a lower alkyl alcohol, wherein alkyl is as herein defined at a temperature of about 30° to 100°C, preferably about 50° to 80°C, for from about 1 to 12 hours, preferably 4 to 7 hours to produce compound IX.

The deprotected form of compound IX, compound X can be produced by hydrogenating the protected compound with for example, a catalyst, for example palladium on carbon, in an organic solvent such as a lower alkyl alcohol wherein alkyl is as hereion defined, a cyclic ether, such as for example tetrahydrofuran, or a lower alkyl ether, wherein alkyl is as herein defined, such as for example diethyl ether, for a time from about 15 minutes to 2 hours, preferably 15 minutes to 1 hour at a temperature of from about 0° to 80°C, preferably room temperature, preferably 20° to 40°C.

The dioxane ring is then formed in step (d) by reaction of X with a strong base such as for example metal alkoxide where metal is sodium, potassium or lithium, preferably lithium and alkyl is as herein defined, preferably lithium tertiary butoxide in the presence of an inert aprotic solvent such as for example a cyclic ether such as for example tetrahydrofuran or a lower alkyl amide where alkyl is as herein defined such as for example dimethylformamide for a time of from 1 to 6 hours, preferably 1 to 3 hours, at a temperature of from about 25° to 100°C, preferably 50° to 70°C; a metal hydride, where metal is for example, sodium, potassium or lithium, preferably sodium hydride, in the presence of an inert aprotic solvent such as for example a lower alkyl amide where alkyl is as herein defined such as for example demethylformamide for a time of from 1 to 6 hours, preferably 1 to 3 hours, at a temperature of from about 25° to 100°C, preferably 50° to 70°C; or with an alkaline carbonate, where alkaline is for example, sodium, potassium or lithium, preferably potassium carbonate in the presence of an organic solvent, preferably a lower alkyl ketone where alkyl is herein defined such as for example acetone for a time of from 1 to 24 hours, preferably 4 to 8 hours, at about reflux temperature. The resulting compound of formula XI (or I) may be isolated by conventional means.

Reaction Sequence III

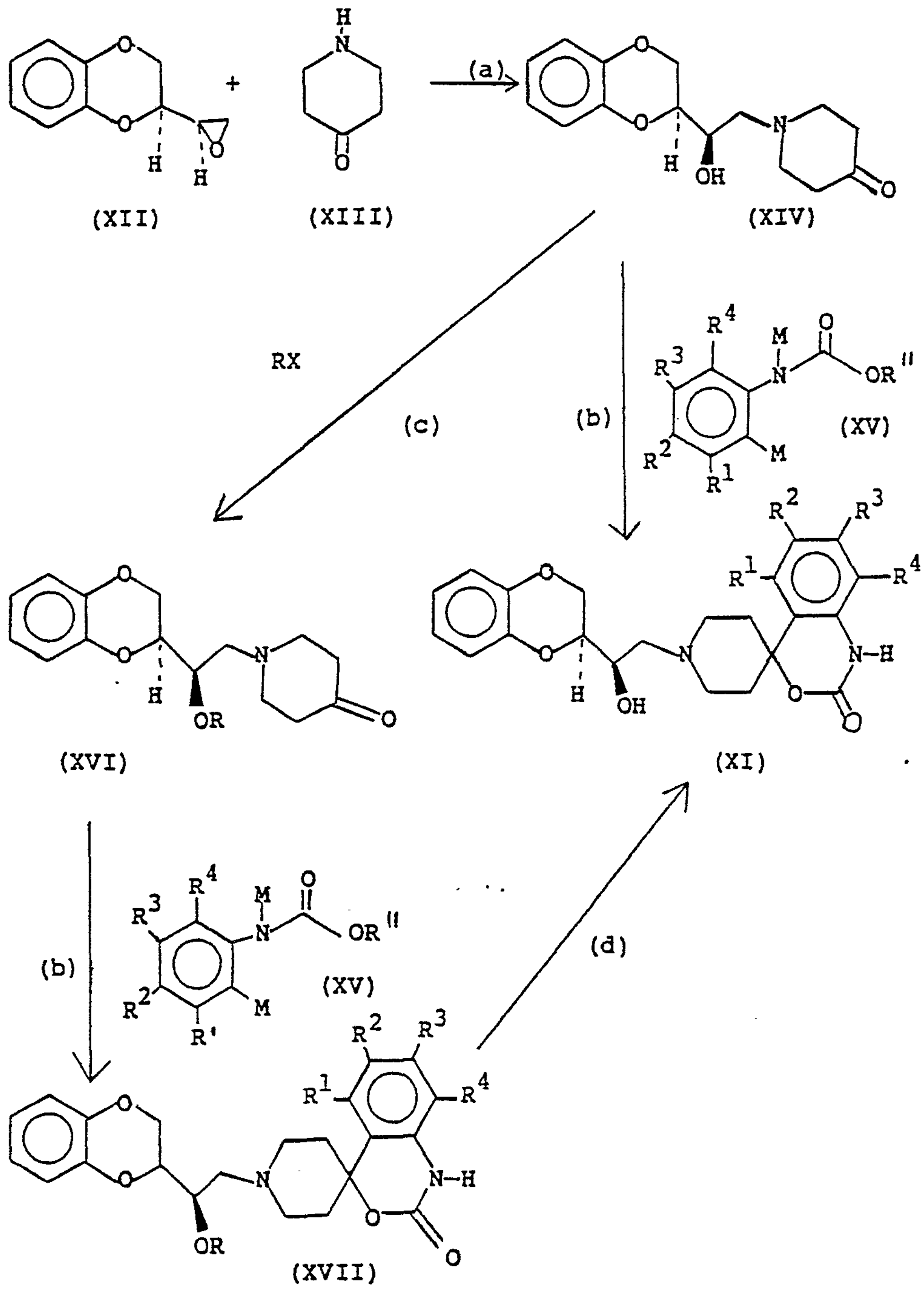


(XII) (XIII) (XIV) (XV) (XVI) (XI) (XV) (XVII)

In carrying out step (a) of Reaction Sequence III compound XII (step (c) of Reaction Sequence I) is reacted with 4-piperidone (XIII) in an organic solvent such as for example a lower alkyl amide where alkyl is as herein defined, such as for example dimethylformamide, a lower alkyl nitrile where alkyl is as herein defined, such as acetonitrile, or a lower alkyl alcohol where alkyl is as herein defined, to form compound XIV.

The alcohol group of compound XIV can be protected with a protecting group using procedures known in the art. Examples of such protecting groups are alkyl or aryl substituted silyl groups, alkyl or aryl substituted acid groups, alkyl or aryl substituted ketone groups, alkyl, substituted alkyl, or aryl group where alkyl and aryl are as herein defined.

In step (b) of Reaction Sequence III the protected or unprotected alcohol or compound XVI or XIV is treated with compound XV (formula III of Reaction Sequence I) wherein $R^1$ represents a tertiary butyl moiety. Compound XV (formula III of Reaction Sequence I) is first treated with a strong base such as a metal hydride or a metal alkide, preferably tertiary butyl lithium, in the presence of an inert aprotic solvent such

as for example a cyclic ether such as tetrahydrofuran, a lower alkyl amide where alkyl is as herein defined such as dimethylformamide, or a lower alkyl sulfoxide where alkyl is as herein defined such as dimethylsulfoxide; preferably a lower alkyl amide and more preferably dimethylformamide. An excess of the base is used, about 1.5—4 moles of base to 1 mole of the compound of formula XV (formula III of Reaction Sequence I), preferably 1.5—2 moles of base. The reaction is kept at a temperature of between about 0° to −80°C, preferably between −20° to −80°C for about 0.5—4 hours, preferably 1—3 hours.

The compound of formula XIV or the protected alcohol of formula XVI is then added dropwise until an approximately stoichiometric amount is added to the reaction mixture as prepared in the previous paragraph, over a period of about 5—60 minutes, preferably about 10 minutes at a temperature of between −20° to −80°C, preferably about −70°C.

The compound of formula XI or XVII may be isolated by procedures known in the art. The compound of formula XVII can be deprotected to form the compound of formula XI by treatment with a deprotecting agent to remove the R group and form the alcohol. The deprotecting group removal can be carried out under for example acid, base, or catalytic conditions, depending on the protecting group used. In general a temperature range of 0° to 50°C will be used.

The alcohol XI can be alkylated using a procedure similar to that described above for protecting the alcohol group.

Isolation and purification of the compounds and intermediates described can be effected, if desired, by any suitable separation or purification procedure such as, for example, filtration, extraction, crystallization, column chromatography, thin-layer chromatography or thick-layer chromatography, or a combination of these procedures. Specific illustrations of suitable separation and isolation procedures can be had by reference to the examples hereinbelow. However, other equivalent separation or isolation procedures could, of course, also be used.

The salt products are also isolated by conventional means. For example, the reaction mixtures may be evaporated to dryness, and the salts can be further purified by conventional methods.

Those compounds of the invention wherein A is 2-(benzodioxan-2-yl)-2-hydroxyethyl, ω-(benzodioxan-2-yl)alkyl, 3-(aryloxy)-2-hydroxypropyl, and ω-aryl-ω-hydroxy alkyl contain at least one chiral center — i.e. the 2-position of the benzodioxanyl moiety and/or the carbon bearing the hydroxy group.

Accordingly, the compounds of the present invention may be prepared in either optically active form or as racemic mixtures. Unless otherwise specified, the compounds described herein are all in the racemic form. However, the scope of the subject invention herein is not to be considered limited to the racemic forms, but to encompass the individual optical isomers of the compounds.

In those embodiments wherein A is 2-(benzodioxan-2-yl)-2-hydroxyethyl, the compounds of the invention may also exist as mixtures of diastereomers (erythro and threo) since both the benzodioxanyl and hydroxyl substituents are present and there are thus two chiral centers. The diastereomers can, of course, be separated by the aforementioned standard separation techniques; however, the scope of the invention herein includes each of the four individually optically pure forms, and also racemic mixtures of each enantiomeric pair, and mixtures of erythro and threo.

A further limitation is that in those embodiments wherein $R^4$ is hydrogen, and $R^1$ and $R^3$ are not identical to each other, mixtures may result from step (a) of Reaction Scheme I, i.e. $R^1$, for example, may end up in either position 5′ or 7′ of the spiro compound. Mixtures of such isomers may then be separated by conventional means.

## Preferred Embodiments

Preferred embodiments of the compounds of the invention are those wherein $R^1$, $R^2$, $R^3$ and $R^4$ are selected from the group consisting of hydrogen, hydroxy, methoxy and halo, and wherein R is hydrogen or methyl.

Particularly preferred are those compounds selected from the group consisting of

1-[2-(benzodioxan-2-yl)-2-hydroxyethyl]-1′-methylspiro[piperidine-4,4′-2′-oxo-3′,1′-benzooxazine];
1-[2-(benzodioxan-2-yl)-2-hydroxyethyl]-spiro[piperidine-4,4′-2′-oxo-3′,1′-benzooxazine];
6′-chloro-1-[2-benzodioxan-2-yl)-2-hydroxyethyl]spiro[piperidine-4,4′-2′-oxo-3′,1′-benzooxazine];
6′-fluoro-1-[2-benzodioxan-2-yl)-2-hydroxyethyl]spiro[piperidine-4,4′-2′-oxo-3′,1′-benzooxazine];
1-(benzodioxan-2-yl-methyl)-spiro[piperidine-4,4′-2′-oxo-3′,1′-benzooxazine];
1-(2-benzodioxan-2-yl)ethyl)-spiro[piperidine-4,4′-2′-oxo-3′,1′-benzooxazine];
5′-hydroxy-1-[2-(benzodioxan-2-yl)-2-hydroxyethyl]spiro[piperidine-4,4′-2′-oxo-3′,1′-benzooxazine];
5′-methoxy-1-[2-(benzodioxan-2-yl)-2-hydroxyethyl]spiro[piperidine-4,4′-2′-oxo-3′,1′-benzooxazine];
1-(2-(2,4-dimethoxyphenoxy)ethyl)-spiro[piperidine-4,4′-2′-oxo-3′,1′-benzooxazine];
1-(2-phenoxyethyl)-spiro[piperidine-4,4′-2′-oxo-3′,1′-benzooxazine];
1-[3-(2-cyanophenoxy)-2-hydroxypropyl]-spiro[piperidine-4,4′-2′-oxo-3′,1′-benzooxazine];
1-[3-phenoxy-2-hydroxypropyl]spiro[piperidine-4,4′-2′-oxo-3′,1′-benzooxazine];
7′-fluoro-1-(3-(2-methoxyphenoxy)-2-hydroxypropyl)spiro[piperidine-4,4′-2′-oxo-3′,1′-benzooxazine];
1-[3-(2-methoxyphenoxy)-2-hydroxypropyl]-spiro[piperidine-4,4′-2′-oxo-3′,1′-benzooxazine];
1-[2-phenyl-2-oxoethyl]-spiro[piperidine-4,4′-2′-oxo-3′,1′-benzooxazine];
1-[4-(4-fluorophenyl)4-oxo-n-butyl]-spiro[piperidine-4,4′-2′-oxo-3′,1′-benzooxazine];
1-[2-phenyl-2-hydroxyethyl]-spiro[piperidine-4,4′-2′-oxo-3′,1′-benzooxazine];

1-[2-(4-hydroxy-3-methoxycarbonylphenyl)-2-hydroxyethyl]-spiro[piperidine-4,4'-2'-oxo-3',1'-benzooxazine];

1-[2-(3-methanesulfamidophenyl)ethyl]-spiro-[piperidine-4,4'-2'-oxo-3',1'-benzooxazine];

1-[2-phenylethyl]spiro[piperidine-4,4'-2'-oxo-3',1'-benzooxazine];

1-(2,6-dichlorobenzyl)-spiro[piperidine-4,4'-2'-oxo-3',1'-benzooxazine].

Utility and Administration

The compounds of the invention lower blood pressure in standard tests, and, accordingly, are useful in ameliorating essential hypertension. These comnpounds, when injected intravenously or intraarterially into the CNS of cats, exhibit an activity similar to clonidine, a well-known $\alpha_2$-antagonist. Additionally, these compounds have been shown to relieve hypertension in spontaneously hypertensive rats (SHR)s when administered orally.

Accordingly, two other aspects of the invention relate to pharmaceutical compositions containing the compounds of this invention, and to methods of ameliorating hypertension using said compounds and said compositions. Administration of the active compounds and salts described herein can be via any of the accepted modes of administration for antihypertensive agents. These methods include oral and intravenous modes, preferably oral administration. Intravenous administration would preferably be reserved for crisis situations, wherein the subject is unable to swallow or administer the medication to himself.

Depending on the intended mode, the compositions may be in the form of solid, semi-solid or liquid dosage forms, such as, for example, tablets, pills, capsules, powders, liquids or suspensions, preferably in unit dosage forms suitable for single administration of precise dosages. The compositions may include a conventional pharmaceutical carrier or excipient and an active compound of Formula I or the pharmaceutically acceptable salts thereof and, in addition, may include other medicinal agents, pharmaceutical agents, carriers or adjuvants.

The amount of active compound administered will, of course, be dependent on the subject being treated, the severity of the affliction, the manner of administration and the judgement of the prescribing physician. However, an effective dosage is in the range of about 0.0001 to 50 mg/kg/day, preferably 0.001 to 10 mg/kg/day if taken orally. For an average 70 kg human, this would amount to 0.007 to 3500 mg per day, or preferably 0.07 to 700 mg/day, if taken orally.

For solid compositions, conventional non-toxic solid carriers include, for example, pharmaceutical grades of mannitol, lactose, starch, magnesium stearate, sodium saccharin, talcum, cellulose, glucose, sucrose, magnesium carbonate, and the like may be used. The active compound as defined above may be formulated as suppositories using, for example, polyalkylene glycols, for example, propylene glycol, as the carrier. Liquid pharmaceutically administerable compositions can, for example, be prepared by dissolving, dispersing, etc. an active compound as defined above and optional pharmaceutical adjuvants in a carrier, such as, for example, water, saline, aqueous dextrose, glycerol or ethanol, to thereby form solution or suspension. If desired, the pharmaceutical composition to be administered may also contain minor amounts of non toxic auxiliary substances such as wetting or emulsifying agents, pH buffering agents, for example, sodium acetate, sorbitan monolaurate, triethanolamine sodium acetate, sorbitan monolaurate or triethanolamine oleate. Actual methods of preparing such dosage forms are known, or will be apparent, to those skilled in this art; for example, see *Remington's Pharmaceutical Sciences,* Mack Publishing Company, Easton, Pennsylvania, 15th Edition, 1975. The composition or formulation to be administered will, in any event, contain a quantity of the active compound(s) in an amount effective to alleviate the symptoms of the subject being treated.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium, carbonate, and the like. Such compositions take the form of solutions, suspensions, tablets, pills, capsules, powders, sustained release formulations and the like. Such compositions may contain 0.1%—95% active ingredient, preferably 1—70%.

For intravenous injections, the compound is dissolved in aqueous medium, buffered to the proper pH and formed to control isotonicity for injection.

The following examples serve to illustrate the invention.

PREPARATION 1

Spiro[piperidine-4,4'-2'-oxo-3',1'-benzooxazine]

A 2.0 M solution of 198 ml *t*-butyllithium n-pentane was added slowly to a −78°C solution containing 34.7 g of N-*t*-butoxycarbonylaniline in 250 ml of THF. After 15 minutes at −78°C, the solution was allowed to warm to −20°C where it was maintained for 2.5 hours. The solution was again cooled to −78°C and a solution of 34.6 g of N-*t*-butoxycarbonyl-4-piperidone in 100 ml THF was added. The mixture was allowed to warm to room temperature and was stirred overnight. Ether (250 ml) was added and the mixture was washed with 5% aqueous hydrochloric acid, water, and brine, dried over $Na_2SO_4$, and evaporated. Chromatography of the residue on silica gel with 40% ethyl acetate-hexane afforded 25.7 g of white solid, t-butoxycarbonyl-protected-spiro[piperidine-4,4'-2'-oxo-3',1'-benzooxazine], mp 83—85°C.

A solution of 20 g of the above compound in 150 ml of methylene chloride and 50 ml of trifluoroacetic acid was stirred for 1 hour at 25°C. Evaporation and treatment of the residue with dilute aqueous sodium hydroxide gave a precipitate, which was filtered to give 11.6 g of tan solid, spiro[piperidine-4,4′-2′-oxo-3′,1′-benzooxazine], m.p. 200—205°C (dec.).

B. Similarly, following the procedure in Part A of this Preparation, but substituting for N-t-butoxycarbonyl aniline:

t-butoxycarbonyl-4-methylaniline;
t-butoxycarbonyl-3,5-diethoxyaniline;
t-butoxy carbonyl-2-chloroaniline;
t-butoxycarbonyl-3-fluoroaniline;
t-butoxycarbonyl-4-fluoroaniline;
t-butoxycarbonyl-4-chloroaniline;

one obtains

6′-methyl-spiro[piperidine-4,4′-2′-oxo-3′,1′-benzooxazine];
5′,7′-diethoxy-spiro[piperidine-4,4′-2′-oxo-3′,1′-benzooxazine];
8′-chloro-spiro[piperidine-4,4′-2′-oxo-3′,1′-benzooxazine];
5′-fluoro-spiro[piperidine-4,4′-2′-oxo-3′,1′-benzooxazine];
6′-fluoro-spiro[piperidine-4,4′-2′-oxo-3′,1′-benzooxazine];
6′-chloro-spiro[piperidine-4,4′-2′-oxo-3′,1′-benzooxazine].

## PREPARATION 2

1′-Methyl-spiro[piperidine-4,4′-2′-oxo-3′,1′-benzooxazine]

A. 1.2 g of 1-t-butoxycarbonyl-spiro[piperidine-4,4′-2′-oxo-3′,1′-benzooxazine] in 25 ml of dimethylformamide was treated with 0.3 g of 50% sodium hydride dispersion in oil and the mixture was stirred for 1 hour at 25°C. Methyl iodide (0.3 ml) was added and stirring was continued for 1 hour. The solution was poured into water, extracted with ethyl acetate, and the extract was evaporated to an oil which was triturated with hexane. The gummy residue was dissolved in 20 ml. of dichloromethane and 2.7 ml of trifluoroacetic acid and the solution was stirred for 2 hours. The solution was washed with dilute sodium bicarbonate and evaporated to afford 0.6 g of 1′-methyl-spiro[piperidine-4,4′-2′-oxo-3′,1′-benzooxazine], m.p. 130—132°C.

B. Similarly, following the procedure in Part A of this Preparation, but substituting for methyl iodide ethyl iodide, i-propyl iodide or n-butyl iodine, one obtains

1′-ethyl-spiro[piperidine-4,4′-2′-oxo-3′,1′-benzooxazine];
1′-i-propyl-spiro[piperidine-4,4′-2′-oxo-3′,1′-benzooxazine];
1′-n-butyl-spiro[piperidine-4,4′-2′-oxo-3′,1′-benzooxazine].

## Example 1

Erythro-1′-(2-benzodioxan-2-yl)-2-hydroxyethyl)spiro[piperidine-4,4′-2′-oxo-3′,1′-benzooxazine]

A. A mixture of 2.2 g of spiro[piperidine-4,4′-2′-oxo-3′,1′-benzooxazine] and 1.8 g of *erythro*-2-epoxybenzodioxane in 30 ml of methanol and 60 ml of toluene was refluxed for 3 hours. The mixture was cooled and filtered to afford 2.7 g of the free base, *erythro*-1′-(2-(benzodioxan-2-yl)-2-hydroxyethyl)spiro-[piperidine-4,4′-2′-oxo-3′,1′-benzooxazine] m.p. 242—243°C.

The hydrochloride salt was prepared by acidifying a methanol solution of the free base with HCl and precipitation with ether. Recrystallization from isopropanol gave the *erythro*-HCl salt, mp 275—277°C.

B. Similarly, following the procedure in Part A of this Example and substituting for spiro[piperidine-4,4′-2′-oxo-3′,1′-benzooxazine] the compounds listed in part B of preparation 1, and Parts A and B of Preparation 2, one obtains:

1-(2-benzodioxan-2-yl)-2-hydroxyethyl)-6′-methylspiro[piperidine-4,4′-2′-oxo-3′,1′-benzooxazine];

1-(2-benzodioxan-2-yl)-2-hydroxyethyl)-5′,7′-diethoxy-spiro[piperidine-4,4′-2′-oxo-3′,1′-benzooxazine];

1-(2-(benzodioxan-2-yl)-2-hydroxyethyl)-8′-chloro-spiro[piperidine-4,4′-2′-oxo-3′,1′-benzooxazine];

1-(2-(benzodioxan-2-yl)-2-hydroxyethyl)-5′-fluorospiro[piperidine-4,4′-2′-oxo-3′,1′-benzooxazine];

2-(benzodioxan-2-yl)-2-hydroxyethyl)-6′-fluoro-spiro[piperidine-4,4′-2′-oxo-3′,1′-benzooxazione]; m.p. 166—168° as the hydrochloride.

2-(benzodioxan-2-yl)-2-hydroxyethyl)-6′-chloro-spiro[piperidine-4,4′-2′-oxo-3′,1′-benzooxazine]; m.p. 165°—168° as the hydrochloride;

1-(2-(benzodioxan-2-yl)-2-hydroxyethyl)-1′-methylspiro[piperidine-4,4′-2′-oxo-3′,1′-benzooxazine] m.p. 140—142° as the hydrochloride;

1-(2-(benzodioxan-2-yl)-2-hydroxyethyl)-1′-ethyl-spiro[piperidine-4,4′-2′-oxo-3′,1′-benzooxazione] m.p. 217—220°C as the hydrochloride;

1-(2-(benzodioxan-2-yl)-2-hydroxyethyl)-1′-n-propyl-spiro[piperidine-4,4′-2′-oxo-3′,1′-benzooxazine];

1-(2-(benzodioxan-2-yl)-2-hydroxyethyl)-1′-n-butyl-spiro[piperidine-4,4′-2′-oxo-3′,1′-benzooxazine].

C. Substituting for *erythro*-2-epoxybenzodioxane into the procedure to Part A, the corresponding *threo,* or *erythro* mixture one obtains the corresponding *threo*-2-(benzodioxan-2-yl)-2-hydroxyethyl-spiro[piperidine-4,4′-2′-oxo-3′,1′-benzooxazine] and *erythro/threo*-2-(benzodioxan-2-yl)-2-hydroxyethyl-

spiro[piperidine-4,4'-2'-oxo-3',1'-benzooxazine], m.p. 210—212° as the free base and 173—175° as the HCl salt.

D. Substituting for *erythro*-2-epoxybenzodioxane into the procedure of Part A of this Example:

3-phenoxypropyl-1,2-epoxide;
3-(4-methylphenoxy)propyl-1,2-epoxide;
3-(2,6-difluorophenoxy)propyl-1,2-epoxide;

one obtains

1-[3-phenoxy-2-hydroxypropyl]-spiro[piperidine-4,4'-2'-oxo-3',1'-benzooxazine], m.p. 263—265° as the HCl salt;
1-[3-(4-methylphenoxy)-2-hydroxypropyl]-spiro[piperidine-4,4'-2'-oxo-3',1'-benzooxazine];
1-[3-(2,6-difluorophenoxy)-2-hydroxypropyl]-spiro[piperidine-4,4'-2'-oxo-3',1'-benzooxazine].

Example 2

1-(2-benzodioxan-2-yl)ethyl)-spiro[piperidine-4,4'-2'-oxo-3',1'-benzooxazine] and its salt

A. A solution of 1.4 g of the spiro[piperidine-4,4'-2'-oxo-3',1'-benzooxazine and 1.25 g of 2-(benzodioxan-2-yl)ethyl bromide in 40 ml of dimethylformamide and 5 ml of triethylamine was heated at 60°C for 5 hours. The mixture was poured into water and extracted with dichloromethane. The extract was washed with water two times, dried, and evporated to afford 0.8 g of the free base, 1-(2-(benzodioxan-2-yl)ethyl)-spiro-[piperidine-4,4'-2'-oxo-3',1'-benzooxazine]; m.p. 65—70°C. The hydrochloride salt, prepared in the usual way had m.p. 238—240°C.

B. Similarly, following the procedure in Part A of this Example but substituting for 2-(benzodioxan-2-yl)ethyl bromide:

benzodioxan-2-ylmethyl bromide;
3-benzodioxan-2-yl)-n-propyl bromide; and
4-(benzodioxan-2-yl)-n-butyl bromide,

one obtains

1-(benzodioxan-2-yl)-methyl-spiro[piperidine-4,4'-2'-oxo-3',1'-benzooxazine]; m.p. 250—252°, as the hydrochloride;
1-[3-(benzodioxan-2-yl)-n-propyl]-spiro[piperidine-4,4'-2'-oxo3',1'-benzooxazine];
1-[4-(benzodioxan-2-yl)-n-butyl]-spiro[piperidine-4,4'-2'-oxo-3',1'-benzooxazine];

and their salts.

C. Similarly, substituting for 2-(benzodioxan-2-yl)-ethyl bromide into the procedure of Part A of this Example:

3-phenoxypropyl bromide;
3-(4-chlorpohenoxy)propyl bromide;
2-(2,4-dimethylphenoxy)ethyl bromide;
3-(3,5-dibromophenoxy)-n-propyl bromide, or
4-(4-cyanophenoxy)-n-butyl bromide;

one obtains

1-(3-phenoxypropyl)-spiro[piperidine-4,4'-2'-oxo-3',1'-benzooxazine] m.p. 250—252°C as the hydrochloride;
1-(3-4-chlorophenoxy)propyl-spiro[piperidine-4,4'-2'-oxo-3',1'-benzooxazine];
1-(2-(2,4-dimethylphenoxy)ethyl)-spiro[piperidine-4,4'-2'-oxo-3',1'-benzooxazine];
(1-3-(3,5-dibromophenoxy)-n-propyl-spiro[piperidine-4,4'-2'-oxo-3',1'-benzooxazine];
1-(4-(4-cyanophenoxy)-n-butyl)-spiro[piperidine-4,4'-2'-oxo-3',1'-benzooxazine].

Example 3

1-(2-phenyl-2-oxoethyl)spiro[piperidine-4,4'-2'-oxo-3',1'-benzooxazine] and its salt

A solution of 1.4 g of spiro[piperidine-4,4'-2'-oxo-3',1'-benzooxazine] and 1.6 g of phenylacetyl bromide in 10 ml of dimethylformamide, 20 ml of acetonitrile, and 7 ml of triethylamine was stirred for 30 minutes at 25°C. Filtration gave 1.3 g of the free base 1-(2-phenyl-α-oxoethyl)-spiro[piperidine-4,4'-2'-oxo-3',1'-benzooxazine], m.p. 190—195°C. The hydrochloride salt had a m.p. 185—189°C.

Example 4

1-(2-phenyl-2-hydroxyethyl)-spiro[piperidine-4,4'-2'-oxo-3',1'-benzooxazine] and its salt

The above ketone (1.25g of free base) was hydrogenated in 75 ml of methanol with 0.5g of 10% palladium on carbon at 45 psi for 12 hours. Filtration and evaporation gave 1.2 g of the alcohol, 1-(2-phenyl-2-hydroxyethyl)spiro[piperidine-4,4'-2'-oxo-3',1'-benzooxazine] m.p. 239—240°C. The hydrochloride salt had m.p. 275—278°C.

Example 5

Preparation of Other Compounds of the Invention

A. Using the procedure in Preparation 1, followed by that outlined in Example 1, the following compounds are prepared:

1-[3-(2-cyanophenoxy)-2-hydroxypropyl]-spiro[piperidine-4,4'-2'-oxo-3',1'-benzooxazine], m.p. 237—238° as the hydrochloride;

1-[3-phenoxy-2-hydroxypropyl]-spiro[piperidine-4,4'-2'-oxo-3',1'-benzooxazine], m.p. 263—265°C as the hydrochloride;

1-[3-(2-methoxyphenoxy)-2-hydroxypropyl]-7'-fluoro-spiro[piperidine-4,4'-2'-oxo-3',1'-benzooxazine], m.p. 120—121° as the hydrochloride;

1-[3-(2-methoxyphenoxy)-2-hydroxypropyl]-spiro[piperidine-4,4'-2'-oxo-3',1'-benzooxazine], m.p. 110—115° as the hydrochloride;

1-[2-(benzodioxan-2-yl)-2-hydroxyethyl]-5'-hydroxy-spiro[piperidine-4,4'-2'-oxo-3',1'-benzooxazine];

1-[2-(benzodioxan-2-yl)-2-hydroxyethyl]-5'-methoxy-spiro[piperidine-4,4'-2'-oxo-3',1'benzooxazine], m.p. 232-235° as the hydrochloride;

1-[3-(4-chlorophenoxy)-2-hydroxypropyl]-spiro[piperidine-4,4'-2'-oxo-3',1'-benzooxazine], m.p. 246—248°C as the hydrochloride; and

1-[3-(4-methoxyphenoxy)-2-hydroxypropyl]-spiro[piperidine-4,4'-2'-oxo-3',1'-benzooxazine], m.p. 230—231°C as the hydrochloride.

B. Using the procedure in Preparation 1, followed by that outlined in Example 2, the following are prepared.

1-[2-(2,6-dimethoxyphenoxy)ethyl]spiro[piperidine-4,4'-2'-oxo-3',1'-benzooxazine], m.p. 243—245°C as the hydrochloride;

1-[2-phenoxyethyl]-spiro[piperidine-4,4'-2'-oxo-3',1'-benzooxazine], m.p. 247—250° as the hydrochloride;

1-[2,6-dichlorobenzyl]-spiro[piperidine-4,4'-2'-oxo-3',1'-benzooxazine], m.p. 241—245° as the hydrochloride;

1-[2-phenylethyl]-spiro[piperidine-4,4'-2'-oxo-3',1'-benzooxazine], m.p. 276—280° as the hydrochloride.

1-[2-(2-methoxyphenoxy)ethyl]-spiro[piperidine-4,4'-2'-oxo-3',1'-benzooxazine], m.p. 151—152°C as the hydrochloride; and

1-[3-(2-methoxyphenoxy)propyl]-spiro[piperidine-4,4'-2'-oxo-3',1'-benzooxazine], m.p. 217—220°C as the hydrochloride.

C. Using the procedure in preparation 1, followed by that outlined in Example 3, the following are prepared:

1-[2-phenyl-2-oxoethyl]-spiro[piperidine-4,4'-2'-oxo-3',1'-benzooxazine], m.p. 190—195°; 185—189° as the hydrochloride;

1-[4-(4-fluorophenyl)-4-oxo-n-butyl]-spiro[piperidine-4,4'-2'-oxo-3',1'-benzooxazine], m.p.225—228° as the hydrochloride.

D. Using the procedure in Preparation 1, followed by that outlined in Example 3, followed by that in Example 4, the following are prepared:

1-[2-phenyl-2-hydroxyethyl]-spiro[piperidine-4,4'-2'-oxo-3',1'-benzooxazine], m.p. 239—240°; 275—278° as the hydrochloride;

1-[2-(3-methoxycarbonyl-4-hydroxyphenyl)-2-hydroxyethyl-spiro[piperidine-4,4'-2'-oxo-3',1'-benzooxazine], m.p. 217—219° as the hydrochloride; and

1-[2-(3-methanesulfamidophenyl)-2-hydroxyethyl]-spiro[piperidine-4,4'-2'-oxo-3',1'-benzooxazine], m.p. 186—187° as the hydrochloride.

## Example 6
## Conversion of Free Base to Salt

A. Excess 3% hydrogen chloride in methanol is added to a solution of 1.0 g 1-[2-(benzodioxan-2-yl)-2-hydroxyethyl]-spiro[piperidine-4,4'-2'-oxo-3',1'-benzooxazine], in 20 ml methanol. Diethyl ether is added until precipitation is complete. The product hydrochloride is filtered, washed with ether, air dried and recrystallized.

B. In a similar manner, all compounds of Formula I in free base form may be converted to the acid addition salts by treatment with the appropriate acid, for example, hydrochloric acid, hydrobromic acid, sulfuric acid, nitric acid, phosphoric acid, acetic acid, propionic acid, glycolic acid, pyruvic acid, oxalic acid, malonic acid, succinic acid, malic acid, maleic acid, fumaric acid, tartaric acid, citric acid, benzoic acid, cinnamic acid, mandelic acid, methansulfonic acid, ethanesulfonic acid, p-toluenesulfonic acid, and the like.

## Example 7
## Conversion of Salt to Free Base

1.0 g of 1-[2-(benzodioxan-2-yl)-2-hydroxyethyl]-spiro[piperidine-4,4'-2'-oxo-3',1'-benzooxazine]. Hydrochloride acid suspended in 50 ml of ether is stirred with excess dilute aqueous potassium carbonate solution until the salt is completely dissolved. The organic layer is then separated, washed twice with water, dried over magnesium sulfate and evaporated to yield 1-[2-(benzodioxan-2-yl)-2-hydroxyethyl]-spiro[piperidine-4,4'-2'-oxo-3',1'-benzooxazine] as the free base.

Example 8

Direct interchange of acid addition salts

1-[2-(benzodioxan-2-yl)-2-hydroxyethyl]-spiro[piperidine-4,4'-2'-oxo-3',1'-benzooxazine] acetate (1.0 g) is dissolved in 50 ml 50% aqueous sulfuric acid, and the solution evaporated to dryness. The product is suspended in ethanol and filtered, air dried and recrystallized from methanol/acetone to yield 1-[2-benzodioxan-2-yl)-2-hydroxyethyl]-spiro[piperidine-4,4'-2'-oxo-3',1'-benzooxazine] as the hydrogen sulfate.

In Examples 9—15, the active ingredient is 1-[2-(benzodioxan-2-yl)-2-hydroxyethyl]-1'-methyl-spiro[piperidine-4,4'-2'-oxo-3',1'-benzooxazine] as its hydrochloride; however, othr comopounds of the invention or their salts may also be used.

Example 9

| Ingredients | Quantity per tablet, mgs. |
|---|---|
| Active ingredient | 25 |
| cornstarch | 20 |
| lactose, spray dried | 153 |
| magnesium stearate | 2 |

The above ingredients are thoroughly mixed and pressed into single scored tablets.

Example 10

| Ingredients | Quantity per tablet, mgs. |
|---|---|
| Active ingredient | 100 |
| lactose, spray-dried | 148 |
| magnesium stearate | 2 |

The above ingredients are mixed and introduced into a hard-shell gelatin capsule.

Example 11

| Ingredients | Quantity per tablet, mgs. |
|---|---|
| Active ingredient | 1 |
| cornstarch | 50 |
| lactose | 145 |
| magnesium sterate | 5 |

The above ingredients are mixed intimately and pressed into single scored tablets.

Example 12

| Ingredients | Quantity per tablet, mgs. |
|---|---|
| Active ingredient | 108 |
| lactose | 15 |
| cornstarch | 25 |
| magnesium stearage | 2 |

The above ingredients are mixed and introduced into a hard-shell gelatin capsule.

Example 13

| Ingredients | Quantity per tablet, mgs. |
|---|---|
| Active ingredient | 150 |
| lactose | 92 |

The above ingredients are mixed and introduced into a hard-shell gelatin capsule.

Example 14

An injectable preparation buffered to a pH of 7 is prepared having the following composition:

| Ingredients | |
|---|---|
| Active ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

Example 15

An oral suspension is prepared having the following composition:

| Ingredients | |
|---|---|
| Active ingredient | 0.1 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co. Trade Mark) | 1.0 g |
| flavoring | 0.035 ml |
| colorings | 0.5 mg |
| distilled water | q.s. to 100 ml |

Example 16

After an initial training period, 24 male, Okamoto-Aoki strain, spontaneously hypertensive rats (Taconic Farms, Germantown, NY) were distributed into six groups of four animals with approximately equal mean systolic blood pressures. The six groups were studied concurrently in a 2-day procedure. Test compounds were randomly assigned to each group. Five groups received test substances, and one control group received vehicle only. On two consecutive mornings, a group of four rats was orally dosed with a test substance that had been dissolved or suspended in water at concentrations such that 0.1 mL of solution was administered per 10 g of body weight. Immediately after dosing on day 2, all 24 rats were put in restrainers and then into a heated chamber (30.0 ± 1.0°C) for 4 h. Systolic blood pressures (tail cuff) were recorded using photoelectric transducers 1, 2, 3, and 4 h after drug administration. The coccygeal arteries of the rats were simultaneously occluded by inflated tail cuffs that were automatically inflated to 300 mmHg and then deflated. Tail pulses were simultaneously recorded, along with a pressure curve on a recorder. Four consecutive (at 3-s intervals) traces were recorded for each rat at each hour after dosing. The systolic pressure was considered to be the pressure at the appearance of the first pulse. The mean systolic pressure of each rat at each observation time in both drug-treated and control groups was calculated. Systolic pressures in the controls varied over the range 180 to 220 mmHg during the 4-h measurement period. The

mean values of the respective drug-treated and control groups were then compared using a 1-tail Student's t test. Statistical significance was considered to be p≤ 0.05.

Table 1 shows the antihypersensitive activity of spiro[4H-3,1-benzoxazin-2(1H)-one-4,4'-piperidines]

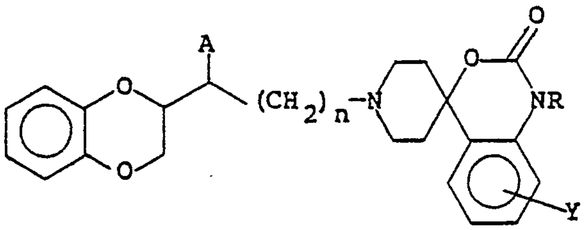

TABLE 1

| A | Compound n | R | Y | Dose mg/kg po | % fall SBP* 1h | 2h | 3h | 4h |
|---|---|---|---|---|---|---|---|---|
| OH[a] | 1 | H | H | 25 | 39 | 33 | 26 | 30 |
| OH[b] | 1 | H | H | 25 | 52 | 42 | 27 | 26 |
| OH[b] | 1 | $CH_3$ | H | 25 | 32 | 40 | 39 | 27 |
| OH[b] | 1 | $CH_2CH_3$ | H | 25 | 40 | 38 | 19 | 29 |
| OH[b] | 1 | H | 5-$OCH_3$ | 12.5 | – | 11 | – | – |
| OH[b] | 1 | H | 6-Cl | 25 | – | 17 | – | – |
| OH[b] | 1 | H | 6-F | 50 | 25 | – | – | – |
| H | 0 | H | H | 50 | 33 | – | – | – |
| H | 1 | H | H | 50 | 53 | 49 | 28 | 35 |

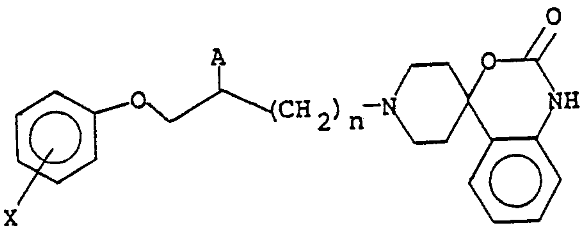

TABLE 2

| Compound X | A | n | Dose mg/kg po | % fall SBP* 1h | 2h | 3h | 4h |
|---|---|---|---|---|---|---|---|
| H | OH | 1 | 50 | 24 | 23 | 15 | 25 |
| 2-$OCH_3$ | OH | 1 | 50 | 38 | 33 | 33 | – |
| 2-CN | OH | 1 | 50 | 24 | 21 | – | 22 |
| 4-Cl | OH | 1 | 25 | 18 | 18 | 23 | 21 |
| 4-$OCH_3$ | OH | 1 | 50 | – | 16 | – | – |
| H | H | 0 | 50 | 26 | 25 | 8 | 16 |
| 2,6-$OCH_3$ | H | 0 | 50 | 23 | 22 | – | – |
| 2-$OCH_3$ | H | 0 | 50 | 15 | 18 | 10 | – |
| H | H | 1 | 50 | 44 | 34 | 29 | 23 |
| 2-$OCH_3$ | H | 1 | 50 | 36 | 37 | 30 | 26 |

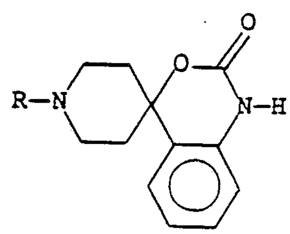

TABLE 3

| Compound R | Dose mg/kg po | % fall SBP* 1h | 2h | 3h | 4h |
|---|---|---|---|---|---|
| $C_6H_5CH_2CH_2$ | 50 | 35 | 34 | 35 | 23 |
| $C_6H_5CHOHCH_2$ | 50 | 43 | 31 | 19 | 25 |
| 4-F $C_6H_4$ CO $CH_2CH_2CH_2$ | 50 | 32 | 33 | – | 25 |

a) 1:1 Erythro-threo mixture.

b) Erythro isomer.

The data represent the percentage decrease in systolic blood pressure for the drug-treated group relative to the value for the untreated control. There were four rats per dosage group. Percentage falls in systolic blood pressure were recorded at the indicated times after dosing on the second day of dosing. Systolic pressures in the controls started at about 200 mmHg (0.26 bar) and varied over the range of 180—229 mmHg (0.24—0.29 bar) during the four-hour measurement period. Values in the table are statistically significant ($p \leq 0.05$) relative to the control values; the horizontal lines denote nonsignificant ($p \geq 0.05$).

**Claims for the Contracting States: BE CH DE FR GB IT LI LU NL SE**

1. A compound of the formula

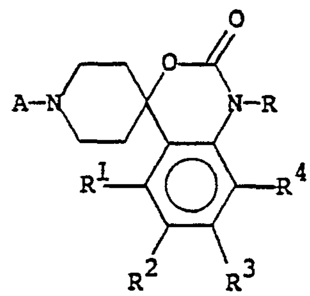

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$ alkoxy or halo;

R is a hydrogen or $C_{1-4}$ alkyl; and

A is selected from:

2-(benzodioxan-2-yl)-2-hydroxyethyl;

ω-(benzodioxan-2-yl)-alkyl (where alkyl is a straight or branched chain hydrocarbon of 1—4 carbons);

3-(aryloxy)-2-hydroxypropyl, wherein aryloxy is phenyloxy optionally substituted by 1, 2, or 3 moities selected from $C_{1-4}$ alkyl, $C_{1-4}$ alkoxy, halo, $C_{1-4}$ alkylsulfamido, ($C_{1-4}$ alkoxy) carbonyl, cyano and trifluoromethyl;

ω-arylalkyl wherein alkyl is as hereinabove defined and wherein aryl is phenyl optionally substituted by 1, 2, or 3 moieties selected from $C_{1-4}$ alkyl, $C_{1-4}$ alkoxy, halo, $C_{1-4}$ alkylsulfamido, ($C_{1-4}$ alkoxy) carbonyl, cyano and trifluoromethyl;

ω-aryl-ω-oxoalkyl (1—4), wherein aryl and alkyl are as hereinabove defined;

ω-aryl-ω-hydroxyalkyl (1—4), wherein aryl and alkyl are as hereinabove defined; and

ω-aryloxyalkyl, wherein alkyl and aryloxy are as herein defined.

2. A compound of claim 1 or a pharmaceutically acceptable acid addition salt thereof, wherein A is 2-(benzodioxan-2-yl)-2-hydroxyethyl or ω-(benzodioxan-2-yl)alkyl.

3. A compound of claim 1 or a pharmaceutically acceptable acid addition salt thereof, wherein A is 3-(aryloxy)-2-hydroxypropyl or ω-aryloxyalkyl.

4. A compound of claim 1 or a pharmaceutically acceptable acid addition salt thereof, wherein A is ω-aryl-ω-oxoalkyl; ω-aryl-ω-hydroxyalkyl or ω-aryloxyalkyl.

5. A compound of claim 1 or a pharmaceutically acceptable acid addition salt thereof, wherein $R^1$, $R^2$, $R^3$ and $R^4$ are each independently hydrogen or halo.

6. A compound of claim 1 or a pharmaceutically acceptable acid addition salt thereof, wherein R is hydrogen or methyl.

7. A compound of claim 2, claim 3 or claim 4 or a pharmaceutically acceptable acid addition salt thereof, wherein $R^1$, $R^2$, $R^3$ and $R^4$ are each independently hydrogen, hydroxy, methoxy or halo; and R is hydrogen or methyl.

8. 1-[2 - (benzodioxan - 2 - yl) - 2 - hydroxyethyl] - spiro[piperidine - 4,4' - 2' - oxo - 3',1' - benzooxazine)],

or a pharmaceutically acceptable and addition salt thereof.

9. A compound of claim 7 selected from

1-[2-(benzodioxan-2-yl)-2-hydroxyethyl]-1'-methyl-spiro[piperidine-4,4'-2'-oxo-3',1'-benzooxazine)],

1-[2-(benzodioxan-2-yl)-2-hydroxyethyl]-6'-chloro-spiro[piperidine-4,4'-2'-oxo-3',1'-benzooxazine)],

1-[2-(benzodioxan-2-yl)-2-hydroxyethyl]-6'-fluoro-spiro[piperidine-4,4'-2'-oxo-3',1'-benzooxazine)],

1-[benzodioxan-2-ylmethyl]-spiro[piperidine-4,4'-2'-oxo-3',1'-benzooxazine],

1-[2-(benzodioxan-2-yl)ethyl]-spiro[piperidine-4,4'-2'-oxo-3',1'-benzooxazine],

1-[2-benzodioxan-2-yl)-2-hydroxyethyl]-5'-hydroxy-spiro[piperidine-4,4'-2'-oxo-3',1'-benzooxazine],

1-[2-(benzodioxan-2-yl)-2-hydroxyethyl]-5'-methoxy-spiro[piperidine-4,4'-2'-oxo-3',1'-benzooxazine],

1-[2-(2,4-dimethoxyphenoxy)ethyl]-spiro[piperidine-4,4'-2'-oxo-3',1'-benzooxazine],

1-[2-phenoxyethyl]-spiro[piperidine-4,4'-2'-oxo-3',1'-benzooxazine],
1-[3-(2-cyanophenoxy)-2-hydroxypropyl]-spiro[piperidine-4,4'-2'-oxo-3',1'-benzooxazine],
1-[3-phenoxy-2-hydroxypropyl]-spiro[piperidine-4,4'-2'-oxo-3',1'-benzooxazine],
7'-fluoro-1-[3-(2-methoxyphenoxy)-2-hydroxypropyl]-spiro[piperidine-4,4'-2'-oxo-3',1'-benzooxazine],
1-[3-(2-methoxyphenoxy)-2-hydroxypropyl]-spiro[piperidine-4,4'-2'-oxo-3',1'-benzooxazine],
1-[2-phenyl-2-oxoethyl]-spiro[piperidine-4,4'-2'-oxo-3',1'-benzooxazine],
1-[4-(4-fluorophenyl)-4-oxo-n-butyl]-spiro[piperidine-4,4'-2'-oxo-3',1'-benzooxazine],
1-[2-phenyl-2-hydroxyethyl]-spiro[piperidine-4,4'-2'-oxo-3',1'-benzooxazine],
1-[3-(3-methanesulfamidophenyl)-2-hydroxyethyl]-spiro[piperidine-4,4'-2'-oxo-3',1'-benzooxazine],
1-[2-(4-hydroxy-3-methoxycarbonylphenyl)-2-hydroxy-ethyl]-spiro[piperidine-4,4'-2'-oxo-3',1'-benzooxazine],
1-[2,6-dichlorobenzyl]-spiro[piperidine-4,4'-2'-oxo-3',1'-benzooxazine],
1-(2-phenylethyl)-spiro[piperidine-4,4'-2'-oxo-3',1'-benzooxazine];
or a pharmaceutically acceptable acid addition salt thereof.

10. A pharmaceutical composition which comprises a therapeutically effective amount of a compound of one of the preceding claims or a pharmaceutically acceptable acid addition salt thereof.

11. A compound of any one of claims 1 to 9 or a pharmaceutically acceptable acid addition salt thereof for use in lowering blood pressure in humans.

12. A process for preparing a compound of claim 1 which process comprises:

a) converting a compound of the formula

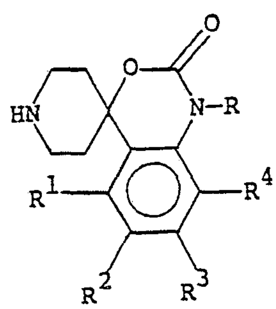

(IV)

wherein $R^1$, $R^2$, $R^3$, and $R^4$, are as herein defined, and R is hydrogen to a compound of formula IV wherein R is $C_{1-4}$ alkyl;

b) treating a compound of formula IV wherein $R^1$, $R^2$, $R^3$, and $R^4$ are as herein defined and R is hydrogen or $C_{1-4}$ alkyl with a compound selected from

A'X and 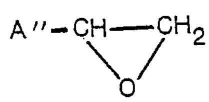

wherein A' is ω-(benzodioxan-2-yl)alkyl, ω-aryloxyalkyl, ω-arylalkyl, or ω-aryl-ω-oxoalkyl; and A'' is benzodioxan-2-yl or aryloxymethyl wherein alkyl and aryl are as herein defined to form a compound of formula I; or

c) converting a compound of formula I wherein R is hydrogen and $R^1$, $R^2$, $R^3$ and $R^4$ have the above meanings to the corresponding product of formula I wherein R is $C_{1-4}$ alkyl; or

d) converting a compound of formula I wherein $R^1$, $R^2$, $R^3$ and $R^4$ are as herein defined, R is hydrogen or $C_{1-4}$ alkyl, and A is ω-aryl-ω-oxyalkyl into the corresponding compound of formula I wherein A is ω-aryl-ω-hydroxyalkyl; or

e) converting the free base of the compound of Formula I to a pharmaceutically acceptable acid addition salt; or

f) converting a salt of the compound of Formula I to a free base; or

g) converting a salt of the compound of Formula I to another salt.

13. A process for preparing a compound of the formula:

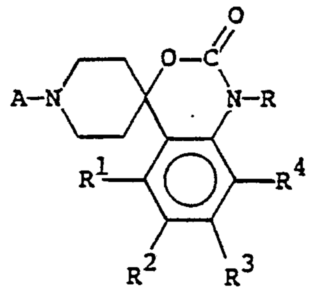

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$alkoxy or halo;

R is hydrogen or $C_{1-4}$ alkyl; and

A is 2-(benzodioxan-2-yl)-2-hydroxyethyl;

which process comprises:

(a) treating a compound of the formula

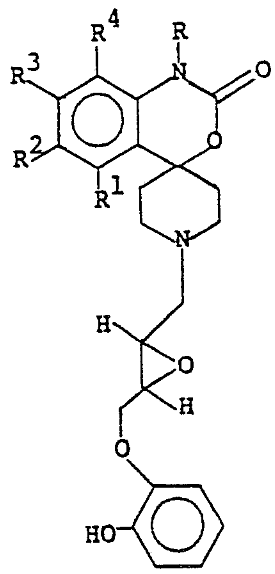

wherein $R^1$, $R^2$, $R^3$, $R^4$, and R are as herein defined with a strong base to form a compound of formula I; or

(b) converting a compound of formula I wherein R is halogen to a compound of formula I wherein R is $C_{1-4}$ alkyl; or

(c) converting the free base of the compound of formula I to a pharmaceutically acceptable acid addition salt; or

(d) converting a salt of the compound of formula I to a free base; or

(e) converting a salt of the compound of formula I to another salt.

14. A process for preparing a compound of the formula:

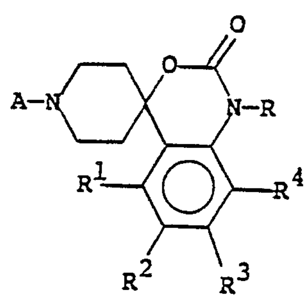

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$ alkoxy or halo;
R is hydrogen; and
A is 2-(benzodioxan-2-yl)-2-hydroxyethyl;
which process comprises:
a) treating a compound of the formula

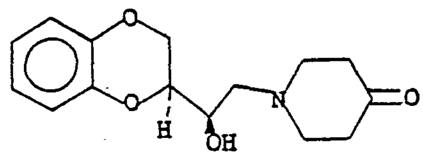

(XIV)

with a compound of the formula

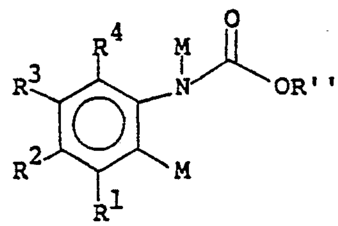

wherein $R^1$, $R^2$, $R^3$, and $R^4$ are as herein defined, M is metal to form a compound of formula I wherein R is hydrogen and $R^1$, $R^2$, $R^3$ and $R^4$ have the above meanings; followed by
b) deprotecting a compound of formula XVII

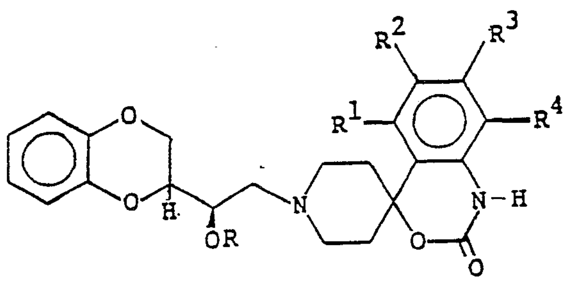

wherein R is an alcohol protecting group and $R^1$, $R^2$, $R^3$, and $R^4$ have the above meanings to obtain a corresponding compound of formula I wherein R is hydrogen, or
c) converting a compound of formula XI

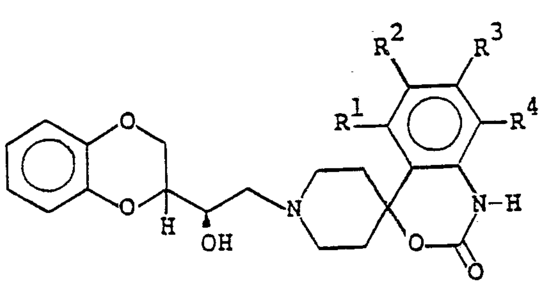

wherein $R'$, $R^2$, $R^3$ and $R^4$ have the above meanings to a corresponding compound of formula I wherein R is $C_{1-4}$ alkyl; or
d) converting the free base of the compound of formula I to a pharmaceutically acceptable acid addition salt; or
e) converting a salt of the compound of formula I to a free base; or
f) converting a salt of the compound of formula I to another salt.

15. A process for preparing a pharmaceutical composition which comprises mixing a product of a process of Claim 12, 13 or 14 with a pharmaceutically acceptable carrier or excipient.

**Claims for the Contracting State: AT**

1. A process for preparing a compound of the formula

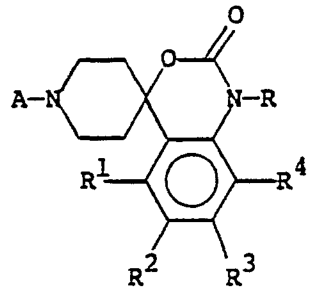

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$ alkoxy or halo;

R is a hydrogen or $C_{1-4}$ alkyl; and

A is selected from:

2-(benzodioxan-2-yl)-2-hydroxyethyl;

ω-(benzodioxan-2-yl)-alkyl (where alkyl is a straight or branched chain hydrocarbon of 1—4 carbons);

3-(aryloxy)-2-hydroxypropyl, wherein aryloxy is phenyloxy optionally substituted by 1, 2, or 3 moieties selected from $C_{1-4}$ alkyl, $C_{1-4}$ alkoxy, halo, $C_{1-4}$ alkylsulfamido, ($C_{1-4}$ alkoxy) carbonyl, cyano and trifluoromethyl;

ω-arylalkyl wherein alkyl is as hereinabove defined and, wherein aryl is phenyl optionally substituted by 1, 2, or 3 moieties selected from $C_{1-4}$ alkyl, $C_{1-4}$ alkoxy, halo, $C_{1-4}$ alkylsulfamido, ($C_{1-4}$ alkoxy) carbonyl, cyano and trifluoromethyl;

ω-aryl-ω-oxoalkyl, wherein alkyl and aryl are as herein defined;

ω-aryl-ω-hydroxyalkyl, wherein alkyl and aryl are as herein defined; and

ω-aryloxyalkyl, wherein alkyl and aryloxy are as herein defined.

which process comprises:

a) converting a compound of the formula

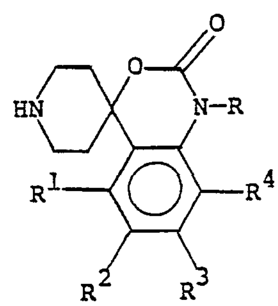

(IV)

wherein $R^1$, $R^2$, $R^3$, and $R^4$, are as herein defined, and R is hydrogen to a compound of formula IV wherein R is $C_{1-4}$ alkyl;

b) treating a compound of formula IV wherein $R^1$, $R^2$, $R^3$, and $R^4$ are as herein defined and R is hydrogen or $C_{1-4}$ alkyl with a compound selected from

A'X and 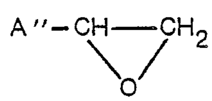

wherein A' is ω-(benzodioxan-2-yl)alkyl, ω-aryloxyalkyl, ω-arylalkyl, or ω-aryl-ω-oxoalkyl; and A'' is benzodioxan-2-yl or aryloxymethyl wherein alkyl and aryl are as herein defined to form a compound of formula I; or

c) converting a compound of formula I wherein R is hydrogen and $R^1$, $R^2$, $R^3$ and $R^4$ have the above

meanings to the corresponding product of formula I wherein R is $C_{1-4}$ alkyl; or

d) converting a compound of formula I wherein $R^1$, $R^2$, $R^3$ and $R^4$ are as herein defined, R is hydrogen or $C_{1-4}$ alkyl, and A is ω-aryl-ω-oxyalkyl into the corresponding compound of formula I wherein A is ω-aryl-ω-hydroxyalkyl; or

e) converting the free base of the compound of Formula I to a pharmaceutically acceptable acid addition salt; or

f) converting a salt of the compound of Formula I to a free base; or

g) converting a salt of the compound of Formula I to another salt.

2. A process for preparing a compound of the formula:

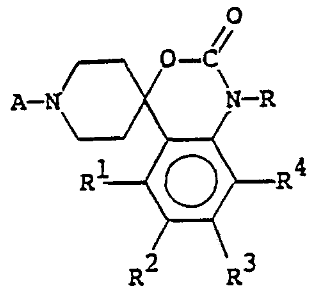

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$ alkoxy or halo;

R is hydrogen or $C_{1-4}$ alkyl; and

A is 2-(benzodioxan-2-yl)-2-hydroxyethyl;

which process comprises:

(a) treating a compound of the formula

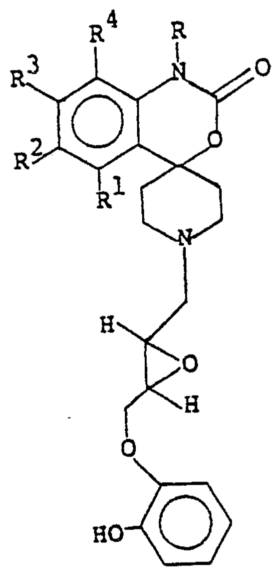

wherein $R^1$, $R^2$, $R^3$, $R^4$, and R are as herein defined with a strong base to form a compound of formula I; or

(b) converting a compound of formula I wherein R is halogen to a compound of formula I wherein R is $C_{1-4}$ alkyl; or

(c) converting the free base of the compound of formula I to a pharmaceutically acceptable acid addition salt; or

(d) converting a salt of the compound of formula I to a free base; or

(e) converting a salt of the compound of formula I to another salt.

3. A process for preparing a compound of the formula:

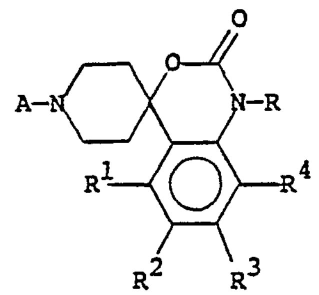

(I)

and the pharmaceutically acceptable acid addition salts thereof, wherein

$R^1$, $R^2$, $R^3$ and $R^4$ are each independently hydrogen, hydroxy, $C_{1-4}$ alkyl, $C_{1-4}$ alkoxy or halo;

R is hydrogen; and

A is 2-(benzodioxan-2-yl)-2-hydroxyethyl;

which process comprises:

a) treating a compound of the formula

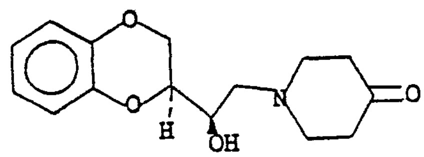

(XIV)

with a compound of the formula

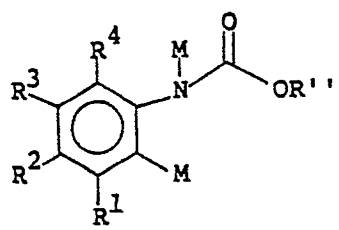

wherein $R^1$, $R^2$, $R^3$, and $R^4$ are as herein defined, M is metal to form a compound of formula I wherein R is hydrogen and $R^1$, $R^2$, $R^3$ and $R^4$ have the above meanings; followed by

b) deprotecting a compound of formula XVII

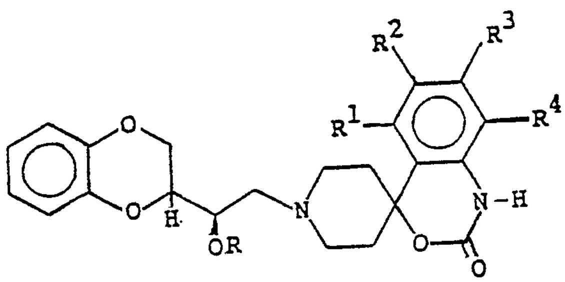

wherein R is an alcohol protecting group and $R^1$, $R^2$, $R^3$, and $R^4$ have the above meanings to obtain a corresponding compound of formula I wherein R is hydrogen, or

c) converting a compound of formula XI

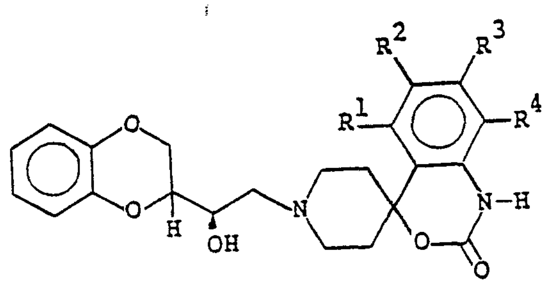

wherein R′, $R^2$, $R^3$ and $R^4$ have the above meanings to a corresponding compound of formula I wherein R is $C_{1-4}$ alkyl; or

d) converting the free base of the compound of formula I to a pharmaceutically acceptable acid addition salt; or

e) converting a salt of the compound of formula I to a free base; or

f) converting a salt of the compound of formula I to another salt.

4. A process of claim 1 wherein A is 2-(benzodioxan-2-yl)-2-hydroxyethyl or ω-(benzodioxan-2-yl)alkyl.

5. The process of claim 1 wherein A is 3-(aryloxy)-2-hydroxypropyl or ω-aryloxyalkyl.

6. The process of claim 1 wherein A is ω-aryl-ω-oxoalkyl; ω-aryl-ω-hydroxyalkyl or ω-aryloxyalkyl.

7. The process of claim 1 wherein $R^1$, $R^2$, $R^3$ and $R^4$ are each independently hydrogen or halo.

8. The process of claim 1 wherein R is hydrogen or methyl.

9. The process of any one of claims 4, 5 and 6 wherein $R^1$, $R^2$, $R^3$ and $R^4$ are each independently hydrogen, hydroxy, methoxy or halo; and R is hydrogen or methyl.

10. The process of claim 1 wheren 1 - [2 - (benzodioxan - 2 - yl) - 2 - hydroxyethyl] - spiro[piperidine - 4,4′ - 2′ - oxo - 3′,1′ - benzooxazine)] or a pharmaceutically acceptable acid addition salt thereof is produced.

11. The process of any one of claims 1 to 9 wherein a compound of formula (I), selected from
1-[2-(benzodioxan-2-yl)-2-hydroxyethyl]-1′-methyl-spiro[piperidine-4,4′-2′-oxo-3′,1′-benzooxazine)],
1-[2-(benzodioxan-2-yl)-2-hydroxyethyl]-6′-chloro-spiro[piperidine-4,4′-2′-oxo-3′,1′-benzooxazine)],
1-[2-(benzodioxan-2-yl)-2-hydroxyethyl]-6′-fluoro-spiro[piperidine-4,4′-2′-oxo-3′,1′-benzooxazine)],
1-[benzodioxan-2-ylmethyl]-spiro[piperidine-4,4′-2′-oxo-3′,1′-benzooxazine],
1-[2-(benzodioxan-2-yl)ethyl]-spiro[piperidine-4,4′-2′-oxo-3′,1′-benzooxazine],
1-[2-(benzodioxan-2-yl)-2-hydroxyethyl]-5′-hydroxy-spiro[piperidine-4,4′-2′-oxo-3′,1′-benzooxazine],
1-[2-(benzodioxan-2-yl)-2-hydroxyethyl]-5′-methoxy-spiro[piperidine-4,4′-2′-oxo-3′,1′-benzooxazine],
1-[2-(2,4-dimethoxyphenoxy)ethyl]-spiro[piperidine-4,4′-2′-oxo-3′,1′-benzooxazine],
1-[2-phenoxyethyl]-spiro[piperidine-4,4′-2′-oxo-3′,1′-benzooxazine],
1-[3-(2-cyanophenoxy)-2-hydroxypropyl]-spiro[piperidine-4,4′-2′-oxo-3′,1′-benzooxazine],
1-[3-phenoxy-2-hydroxypropyl]-spiro[piperidine-4,4′-2′-oxo-3′,1′-benzooxazine],
7′-fluoro-1-[3-(2-methoxyphenoxy)-2-hydroxypropyl]-spiro[piperidine-4,4′-2′-oxo-3′,1′-benzooxazine],
1-[3-(2-methoxyphenoxy)-2-hydroxypropyl]-spiro[piperidine-4,4′-2′-oxo-3′,1′-benzooxazine],
1-[2-phenyl-2-oxoethyl]-spiro[piperidine-4,4′-2′-oxo-3′,1′-benzooxazine],
1-[4-(4-fluorophenyl)-4-oxo-n-butyl]-spiro[piperidine-4,4′-2′-oxo-3′,1′-benzooxazine],
1-[2-phenyl-2-hydroxyethyl]-spiro[piperidine-4,4′-2′-oxo-3′,1′-benzooxazine],
1-[3-(3-methanesulfamidophenyl)-2-hydroxyethyl]-spiro[piperidine-4,4′-2′-oxo-3′,1′-benzooxazine],
1-[2-(4-hydroxy-3-methoxycarbonylphenyl)-2-hydroxy-ethyl]-spiro[piperidine-4,4′-2′-oxo-3′,1′-benzooxazine],
1-[2,6-dichlorobenzyl]-spiro[piperidine-4,4′-2′-oxo-3′,1′-benzooxazine],
1-[2-phenylethyl)-spiro[piperidine-4,4′-2′-oxo-3′,1′-benzooxazine];
or a pharmaceutically acceptable acid addition salt thereof is produced.

12. A process for preparing a pharmaceutical composition which comprises mixing a compound of formula (I) as defined in any of the preceding claims, or a pharmaceutically acceptable acid addition salt thereof, or a product of a process of any one of the preceding claims with a pharmaceutically acceptable carrier or excipient.

**Patentansprüche für die Vertragsstaaten: BE CH DE FR GB IT LI LU NL SE**

1. Eine Verbindung der Formel

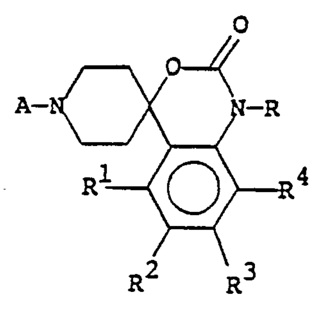

(I)

und die pharmazeutisch annehmbaren Säureadditionssalze derselben worin
$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;
R Wasserstoff oder $C_{1-4}$ Alkyl ist; und

A ausgewählt ist aus:

2-(Benzodioxan-2-yl)-2-hydroxyethyl;

ω-(Benzodioxan-2-yl)-alkyl (wobei Alkyl ein gerad- oder verzweigtkettiger Kohlenwasserstoff mit 1 bis 4 Kohlenstoffatomen ist);

3-(Aryloxy)-2-hydroxypropyl, worin Aryloxy Phenyloxy ist, das wahlweise durch 1, 2 oder 3 Reste, ausgewählt aus $C_{1-4}$ Alkyl, $C_{1-4}$ Alkoxy, Halogen, $C_{1-4}$ Alkylsulfamido, ($C_{1-4}$ Alkoxy)-carbonyl, Cyano und Trifluormethyl, substituiert ist;

ω-Arylalkyl, worin Alkyl wie oben definiert ist und Aryl Phenyl ist, das wahlweise durch 1, 2 oder 3 Reste, ausgewählt aus $C_{1-4}$ Alkyl, $C_{1-4}$ Alkoxy, Halogen, $C_{1-4}$ Alkylsulfamido, ($C_{1-4}$ Alkoxy)-carbonyl, Cyano und Trifluormethyl, substituiert ist;

ω-Aryl-ω-oxoalkyl (1—4), worin Aryl und Alkyl wie oben definiert sind;

ω-Aryl-ω-hydroxyalkyl (1—4), worin Aryl und Alkyl wie oben definiert sind; und

ω-Aryloxyalkyl, worin Alkyl und Aryloxy wie oben definiert sind.

2. Eine Verbindung nach Anspruch 1 oder ein pharmazeutisch annehmbares Säureadditionssalz derselben, worin A 2-(Benzodioxan-2-yl)-2-hydroxyethyl oder ω-(Benzodioxan-2-yl)alkyl ist.

3. Eine Verbindung nach Anspruch 1 oder ein pharmazeutisch annehmbares Säureadditionssalz derselben, worin A 3-(Aryloxy)-2-hydroxypropyl oder ω-Aryloxyalkyl ist.

4. Eine Verbindung nach Anspruch 1 oder ein pharmazeutisch annehmbares Säureadditionssalz derselben, worin A ω-Aryl-ω-oxoalkyl, ω-Aryl-ω-hydroxyalkyl oder ω-Aryloxyalkyl ist.

5. Eine Verbindung nach Anspruch 1 oder ein pharmazeutisch annehmbares Säureadditionssalz derselben, worin $R^1$, $R^2$, $R^3$ und $R^4$ jeweils unabhängig Wasserstoff oder Halogen sind.

6. Eine Verbindung nach Anspruch 1 oder ein pharmazeutisch annehmbares Säureadditionssalz derselben, worin R Wasserstoff oder Methyl ist.

7. Eine Verbindung nach Anspruch 2, 3 oder 4 oder ein pharmazeutisch annehmbares Säureadditionssalz derselben, worin $R^1$, $R^2$, $R^3$ und $R^4$ jeweils unabhängig Wasserstoff, Hydroxy, Methoxy oder Halogen sind und R Wasserstoff oder Methyl ist.

8. 1-[2 - Benzodioxan - 2 - yl) - 2 - hydroxyethyl] - spiro[piperidin - 4,4′ - 2′ - oxo - 3′,1′ - benzooxazin)] oder ein pharmazeutisch annehmbares Säureadditionssalz desselben.

9. Eine Verbindung nach Anspruch 7, ausgewählt aus

1-[2-(Benzodioxan-2-yl)-2-hydroxyethyl]-1′-methyl-spiro[piperidin-4,4′-2′-oxo-3′,1′-benzooxazin)],

1-[2-(Benzodioxan-2-yl)-2-hydroxyethyl]-6′-chlor-spiro[piperidin-4,4′-2′-oxo-3′,1′-benzooxazin)],

1-[2-(Benzodioxan-2-yl)-2-hydroxyethyl]-6′-fluor-spiro[piperidin-4,4′-2′-oxo-3′,1′-benzooxazin)],

1-[Benzodioxan-2-ylmethyl]-spiro[piperidin-4,4′-2′-oxo-3′,1′-benzooxazin],

1-[2-(Benzodioxan-2-yl)-ethyl]-spiro[piperidin-4,4′-2′-oxo-3′,1′-benzooxazin],

1-[2-(Benzodioxan-2-yl)-2-hydroxyethyl]-5′-hydroxy-spiro[piperidin-4,4′-2′-oxo-3′,1′-benzooxazin],

1-[2-(Benzodioxan-2-yl)-2-hydroxyethyl]-5′-methoxy-spiro[piperidin-4,4′-2′-oxo-3′,1′-benzooxazin],

1-[2-(2,4-Dimethoxyphenoxy)-ethyl]-spiro[piperidin-4,4′-2′-oxo-3′,1′-benzooxazin],

1-[2-Phenoxyethyl]-spiro[piperidin-4,4′-2′-oxo-3′,1′-benzooxazin],

1-[3-(2-Cyanphenoxy)-2-hydroxypropyl]-spiro[piperidin-4,4′-2′-oxo-3′,1′-benzooxazin],

1-[3-Phenoxy-2-hydroxypropyl]-spiro[piperidin-4,4′-2′-oxo-3′,1′-benzooxazin],

7′-Fluor-1-[3-(2-methoxyphenoxy)-2-hydroxypropyl]-spiro[piperidin-4,4′-2′-oxo-3′,1′-benzooxazin],

1-[3-(2-Methoxyphenoxy)-2-hydroxypropyl]-spiro[piperidin-4,4′-2′-oxo-3′,1′-benzooxazin],

1-[2-Phenyl-2-oxoethyl]-spiro[piperidin-4,4′-2′-oxo-3′,1′-benzooxazin],

1-[4-(4-Fluorphenyl)-4-oxo-n-butyl]-spiro[piperidin-4,4′-2′-oxo-3′,1′-benzooxazin],

1-[2-Phenyl-2-hydroxyethyl]-spiro[piperidin-4,4′-2′-oxo-3′,1′-benzooxazin],

1-[3-(3-Methansulfamidophenyl)-2-hydroxyethyl]-spiro[piperidin-4,4′-2′-oxo-3′,1′-benzooxazin],

1-[2-(4-Hydroxy-3-methoxycarbonylphenyl)-2-hydroxyethyl]-spiro[piperidin-4,4′-2′-oxo-3′,1′-benzooxazin],

1-[2,6-Dichlorbenzyl]-spiro[piperidin-4,4′-2′-oxo-3′,1′-benzooxazin],

1-(2-Phenylethyl)-spiro[piperidin-4,4′-2′-oxo-3′,1′-benzooxazin],

oder einem pharmazeutisch annehmbaren Säureadditionssalz derselben.

10. Pharmazeutische Zusammensetzung, enthaltend eine therapeutisch wirksame Menge einer Verbindung nach einem der vorhergehenden Ansprüche oder eines pharmazeutisch annehmbaren Säureadditionssalzes derselben.

11. Eine Verbindung nach einem der Ansprüche 1 bis 8 oder ein pharmazeutisch annehmbares Säureadditionssalz derselben zur Verwendung am Menschen zwecks Senkung des Blutdruckes.

12. Verfahren zur Herstellung einer Verbindung nach Anspruch 1, dadurch gekennzeichnet, daß man

a) eine Verbindung der Formel

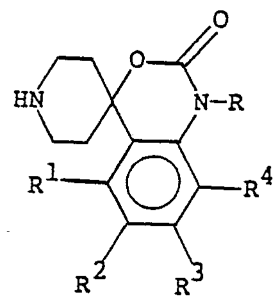

(IV)

worin $R^1$, $R^2$, $R^3$ und $R^4$ wie oben definiert sind und R Wasserstoff ist, in eine Verbindung der Formel IV umwandelt, in welcher R $C_{1-4}$ Alkyl ist;

b) eine Verbindung der Formel IV, in welcher $R^1$, $R^2$, $R^3$ und $R^4$ wie oben definiert sind und R Wasserstoff oder $C_{1-4}$ Alkyl ist, mit einer Verbindung, ausgewählt aus

A'X und 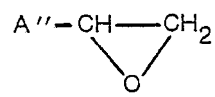

worin A' ω-(benzodioxan-2-yl)-alkyl, ω-Aryloxyalkyl, ω-Arylalkyl oder ω-Aryl-ω-oxyalkyl ist und A'' Benzodioxan-2-yl oder Aryloxymethyl ist, worin Alkyl und Aryl wie oben definiert sind, zur Bildung einer Verbindung der Formel I behandelt; oder

c) eine Verbindung der Formel I, in welcher R Wasserstoff ist und $R^1$, $R^2$, $R^3$ und $R^4$ die obige Bedeuting haben, in das entsprechende Produkt der Formel I umwandelt, in welcher R $C_{1-4}$ Alkyl ist; oder

d) eine Verbindung der Formel I, in welcher $R^1$, $R^2$, $R^3$ und $R^4$ wie oben definiert sind, R Wasserstoff oder $C_{1-4}$ Alkyl ist und A ω-Aryl-ω-oxyalkyl ist, in die entsprechende Verbindung der Formel I umwandelt, in welcher A ω-Aryl-ω-hydroxyalkyl ist; oder

e) die freie Base der Verbindung der Formel I in ein pharmazeutisch annehmbares Säureadditionssalz umwandelt; oder

f) ein Salz der Verbindung der Formel I in eine freie Base umwandelt; oder

g) ein Salz der Verbindung der Formel I in ein anderes Salz umwandelt.

13. Verfahren zur Herstellung einer Verbindung der Formel

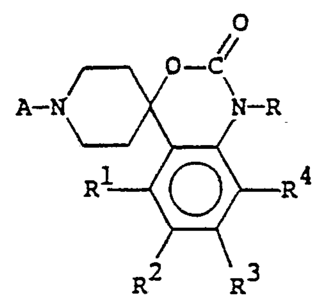

(I)

und der pharmazeutisch annehmbaren Säureadditionssalze derselben, worin

$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;

R Wasserstoff oder $C_{1-4}$ alkyl ist; und

A 2-(Benzodioxan-2-yl)-2-hydroxyethyl ist;

dadurch gekennzeichnet, daß man

a) eine Verbindung der Formel

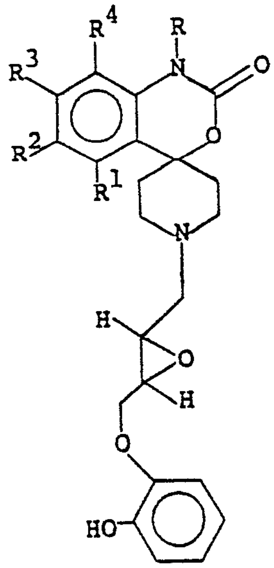

worin $R^1$, $R^2$, $R^3$, $R^4$ und R wie oben definiert sind, mit einer starken Base zur Bildung einer Verbindung der Formel I behandelt; oder

b) eine Verbindung der Formel I, in welcher R Wasserstoff ist, in eine Verbindung der Formel I umwandelt, in welcher R $C_{1-4}$ Alkyl ist; oder

c) die freie Base der Verbindung der Formel I in ein pharmazeutisch annehmbares Säureadditionssalz umwandelt; oder

d) ein Salz der Verbindung der Formel I in eine freie Base umwandelt; oder

e) ein Salz der Verbindung der Formel I in ein anderes Salz umwandelt.

14. Verfahren zur Herstellung einer Verbindung der Formel

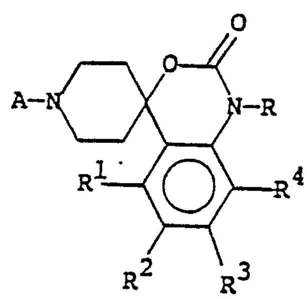

(I)

und der pharmazeutisch annehmbaren Säureadditions salze derselben, worin

$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;

R Wasserstoff ist; und

A 2-(Benzodioxan-2-yl)-2-hydroxyethyl ist;

dadurch gekennzeichnet, daß man

a) eine Verbindung der Formel

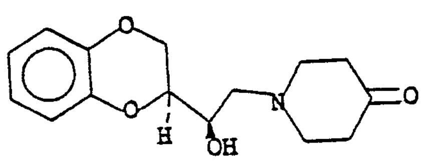

(XIV)

mit einer Verbindung der Formel

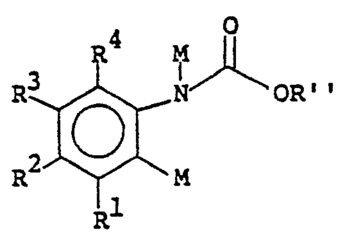

in welcher $R^1$, $R^2$, $R^3$ und $R^4$ wie oben definiert sind und M Metall ist, zur Bildung einer Verbindung der Formel I behandelt, in welcher R Wasserstoff ist und $R^1$, $R^2$, $R^3$ und $R^4$ die obige Bedeutung haben; und anschließend

b) die Schutzgruppen einer Verbindung der Formel XVII

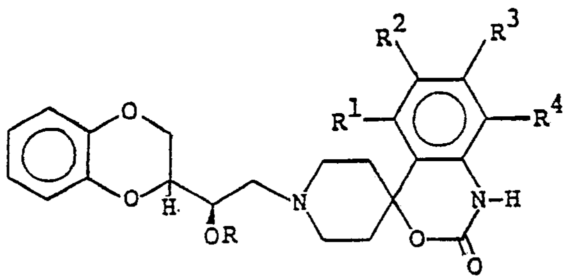

worin R eine Alkoholschutzgruppe ist und $R^1$, $R^2$, $R^3$ und $R^4$ die obige Bedeutung haben, entfernt, um eine entsprechende Verbindung der Formel I zu erhalten, in welcher R Wasserstoff ist; oder

c) eine Verbindung der Formel XI

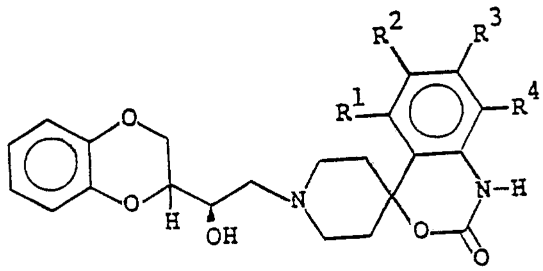

worin $R^1$, $R^2$, $R^3$ und $R^4$ die obige Bedeutung haben, in eine entsprechende Verbindung der Formel I umwandelt, in welcher R $C_{1-4}$ Alkyl ist; oder

d) die freie Base der Verbindung der Formel I in ein pharmazeutisch annehmbares Säureadditionssalz umwandelt; oder

e) ein Salz der Verbindung der Formel I in eine freie Base umwandelt; oder

f) ein Salz der Verbindung der Formel I in ein anderes Salz umwandelt.

15. Verfahren zur Herstellung einer pharmazeutischen Zusammensetzung, dadurch gekennzeichnet, daß man ein Produkt eines Verfahrens von Anspruch 12, 13 oder 14 mit einem pharmazeutisch annehmbaren Träger oder Streckmittel mischt.

**Patentansprüche für den Vertragsstaat: AT**

1. Verfahren zur Herstellung einer Verbindung der Formel

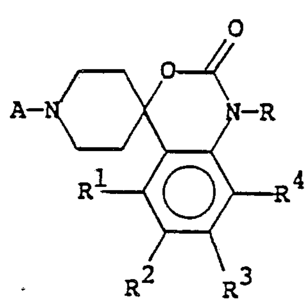

(I)

und der pharmazeutisch annehmbaren Säureadditionssalze derselben worin

$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;

R Wasserstoff oder $C_{1-4}$ Alkyl ist; und

A ausgewählt ist aus:

2-(Benzodioxan-2-yl)-2-hydroxyethyl;

ω-(Benzodioxan-2-yl)-alkyl (wobei alkyl ein gerad- oder verzweigtkettiger Kohlenwasserstoff mit 1 bis 4 Kohlenstoffatomen ist);

3-(Aryloxy)-2-hydroxypropyl, worin Aryloxy Phenyloxy ist, das wahlweise durch 1, 2 oder 3 Reste, ausgewählt aus $C_{1-4}$ Alkyl, $C_{1-4}$ Alkoxy, Halogen, $C_{1-4}$ Alkylsulfamido, ($C_{1-4}$ Alkoxy)-carbonyl, Cyano und Trifluormethyl, substituiert ist;

ω-Arylalkyl, worin Alkyl wie oben definiert ist und Aryl Phenyl ist, das wahlweise durch 1, 2 oder 3 Reste, ausgewählt aus $C_{1-4}$ Alkyl, $C_{1-4}$ Alkoxy, Halogen, $C_{1-4}$ Alkylsulfamido, ($C_{1-4}$ Alkoxy)-carbonyl, Cyano und Trifluoromethyl, substituiert ist;

ω-Aryl-ω-oxoalkyl, worin Aryl und Alkyl wie oben definiert sind;

ω-Aryl-ω-hydroxyalkyl, worin Aryl und Alkyl wie oben definiert sind; und

ω-Aryloxyalkyl, worin Alkyl und Aryloxy wie oben definiert sind,

dadurch gekennzeichnet, daß man

a) eine Verbindung der Formel

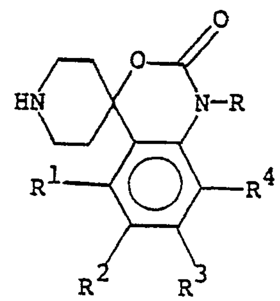

(IV)

worin $R^1$, $R^2$, $R^3$ und $R^4$ wie oben definiert sind und R Wasserstoff ist, in eine Verbindung der Formel IV umwandelt, in welcher R $C_{1-4}$ Alkyl ist;

b) eine Verbindung der Formel IV, in welcher $R^1$, $R^2$, $R^3$ und $R^4$ wie oben definiert sind und R Wasserstoff oder $C_{1-4}$ Alkyl ist, mit einer Verbindung, ausgewählt aus

A'X und 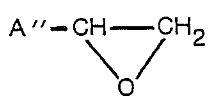

worin A' ω-(benzodioxan-2-yl)-alkyl, ω-Aryloxyalkyl, ω-Arylalkyl oder ω-Aryl-ω-oxyalkyl ist und A'' Benzodioxan-2-yl oder Aryloxymethyl ist, worin Alkyl und Aryl wie oben definiert sind, zur Bildung einer Verbindung der Formel I behandelt; oder

c) eine Verbindung der Formel I, in welcher R Wasserstoff ist und $R^1$, $R^2$, $R^3$ und $R^4$ die obige Bedeutung haben, in das entsprechende Produkt der Formel I umwandelt, in welcher R $C_{1-4}$ Alkyl ist; oder

d) eine Verbindung der Formel I, in welcher $R^1$, $R^2$, $R^3$ und $R^4$ wie oben definiert sind, R Wasserstoff oder $C_{1-4}$ Alkyl ist und A ω-Aryl-ω-oxyalkyl ist, in die entsprechende Verbindung der Formel I umwandelt, in welcher A ω-Aryl-ω-hydroxyalkyl ist; oder

e) die freie Base der Verbindung der Formel I in ein pharmazeutisch annehmbares Säureadditionssalz umwandelt; oder

f) ein Salz der Verbindung der Formel I in eine freie Base umwandelt; oder

g) ein Salz der Verbindung der Formel I in ein anderes Salz umwandelt.

2. Verfahren zur Herstellung einer Verbindung der Formel

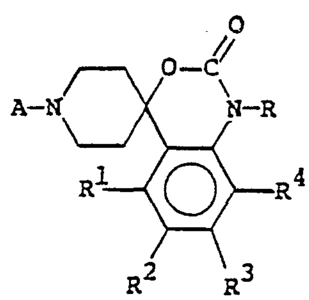

(I)

und der pharmazeutisch annehmbaren Säureadditionssalze derselben, worin

$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;

R Wasserstoff oder $C_{1-4}$ alkyl ist; und

A 2-(Benzodioxan-2-yl)-2-hydroxyethyl ist;

dadurch gekennzeichnet, daß man

a) eine Verbindung der Formel

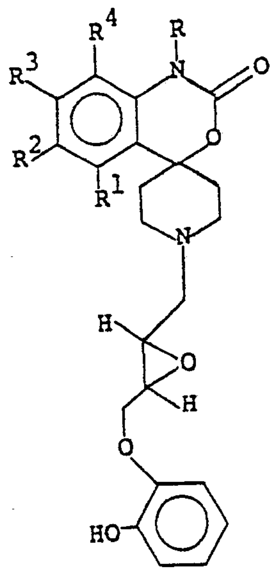

worin $R^1$, $R^2$, $R^3$, $R^4$ und R wie oben definiert sind, mit einer starken Base zur Bildung einer Verbindung der Formel I behandelt; oder

b) eine Verbindung der Formel I, in welcher R Wasserstoff ist, in eine Verbindung der Formel I umwandelt, in welcher R $C_{1-4}$ Alkyl ist; oder

c) die freie Base der Verbindung der Formel I in ein pharmazeutisch annehmbares Säureadditionssalz umwandelt; oder

d) ein Salz der Verbindung der Formel I in eine freie Base umwandelt; oder

e) ein Salz der Verbindung der Formel I in ein anderes Salz umwandelt.

3. Verfahren zur Herstellung einer Verbindung der Formel

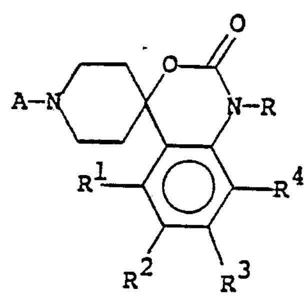

(I)

und der pharmazeutisch annehmbaren Säureadditions salze derselben, worin

$R^1$, $R^2$, $R^3$ und $R^4$ jeweils unabhängig Wasserstoff, Hydroxy, $C_{1-4}$ Alkyl, $C_{1-4}$ Alkoxy oder Halogen sind;

R Wasserstoff ist; und

A 2-(Benzodioxan-2-yl)-2-hydroxyethyl ist;

dadurch gekennzeichnet, daß man

a) eine Verbindung der Formel

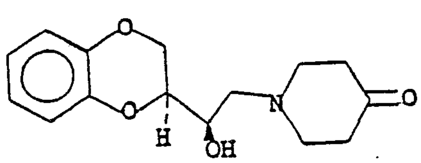

(XIV)

mit einer Verbindung der Formel

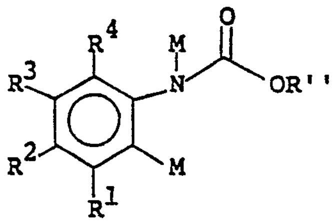

in welcher $R^1$, $R^2$, $R^3$ und $R^4$ wie oben definiert sind und M Metall ist, zur Bildung einer Verbindung der Formel I behandelt, in welcher R Wasserstoff ist und $R^1$, $R^2$, $R^3$ und $R^4$ die obige Bedeutung haben; und anschließend

b) die Schutzgruppen einer Verbindung der Formel XVII

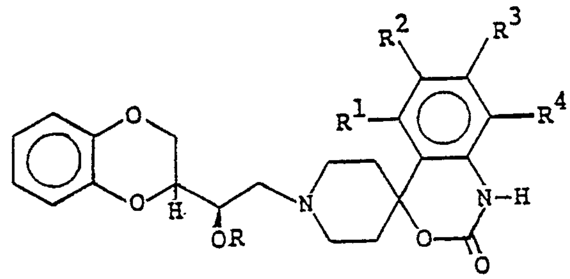

worin R eine Alkoholschutzgruppe ist und $R^1$, $R^2$, $R^3$ und $R^4$ die obige Bedeutung haben, entfernt, um eine entsprechende Verbindung der Formel I zu erhalten, in welcher R Wasserstoff ist; oder

c) eine Verbindung der Formel XI

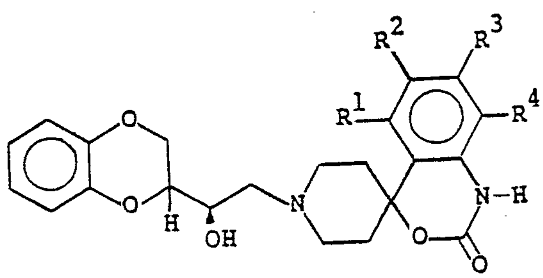

worin $R^1$, $R^2$, $R^3$ und $R^4$ die obige Bedeutung haben, in eine entsprechende Verbindung der Formel I umwandelt, in welcher R $C_{1-4}$ Alkyl ist; oder

d) die freie Base der Verbindung der Formel I in ein pharmazeutisch annehmbares Säureadditionssalz umwandelt; oder

e) ein Salz der Verbindung der Formel I in eine freie Base umwandelt; oder

f) ein Salz der Verbindung der Formel I in ein anderes Salz umwandelt.

4. Verfahren nach Anspruch 1, in welchem A 2-(Benzodioxan-2-yl)-2-hydroxyethyl oder ω-(Benzodioxan-2-yl)-alkyl ist.

5. Verfahren nach Anspruch 1, in welchem A 3-(Aryloxy)-2-hydroxypropyl oder ω-Aryloxyalkyl ist.

6. Verfahren nach Anspruch 1, in welchem A ω-Aryl-ω-oxoalkyl, ω-Aryl-ω-hydroxyalkyl oder ω-Aryloxyalkyl ist.

7. Verfahren nach Anspruch 1, in welchem $R^1$, $R^2$, $R^3$ und $R^4$ jeweils unabhängig Wasserstoff oder Halogen sind.

8. Verfahren nach Anspruch 1, welchem R Wasserstoff oder Methyl ist.

9. Verfahren nach einem der Ansprüche 4, 5 und 6, in welchem $R^1$, $R^2$, $R^3$ und $R^4$ jeweils unabhängig Wasserstoff, Hydroxy, Methoxy oder Halogen sind und R Wasserstoff oder Methyl ist.

10. Verfahren nach Anspruch 1, in welchem 1 - [2 - (Benzodioxan - 2 - yl) - 2 - hydroxyethyl] - spiro[piperidin - 4,4' - 2' - oxo - 3',1' - benzooxazin)] oder ein pharmazeutisch annehmbares Säureadditionssalz desselben hergestellt wird.

11. Verfahren nach einem der Ansprüche 1 bis 9, in welchem eine Verbindung der Formel (I), ausgewählt aus

1-[2-(Benzodioxan-2-yl)-2-hydroxyethyl]-1'-methyl-spiro[piperidin-4,4'-2'-oxo-3',1'-benzooxazin)],

1-[2-(Benzodioxan-2-yl)-2-hydroxyethyl]-6'-chlor-spiro[piperidin-4,4'-2'-oxo-3',1'-benzooxazin)],

1-[2-(Benzodioxan-2-yl)-2-hydroxyethyl]-6'-fluor-spiro[piperidin-4,4'-2'-oxo-3',1'-benzooxazin)],

1-[Benzodioxan-2-ylmethyl]-spiro[piperidin-4,4'-2'-oxo-3',1'-benzooxazin],

1-[2-(Benzodioxan-2-yl)-ethyl]-spiro[piperidin-4,4'-2'-oxo-3',1'-benzooxazin],

1-[2-(Benzodioxan-2-yl)-2-hydroxyethyl]-5'-hydroxy-spiro[piperidin-4,4'-2'-oxo-3',1'-benzooxazin],
1-[2-(Benzodioxan-2-yl)-2-hydroxyethyl]-5'-methoxy-spiro[piperidin-4,4'-2'-oxo-3',1'-benzooxazin],
1-[2-(2,4-Dimethoxyphenoxy)-ethyl]-spiro[piperidin-4,4'-2'-oxo-3',1'-benzooxazin],
1-[2-Phenoxyethyl]-spiro[piperidin-4,4'-2'-oxo-3',1'-benzooxazin],
1-[3-(2-Cyanphenoxy)-2-hydroxypropyl]-spiro[piperidin-4,4'-2'-oxo-3',1'-benzooxazin],
1-[3-Phenoxy-2-hydroxypropyl]-spiro[piperidin-4,4'-2'-oxo-3',1'-benzooxazin],
7'-Fluor-1-[3-(2-methoxyphenoxy)-2-hydroxypropyl]-spiro[piperidin-4,4'-2'-oxo-3',1'-benzooxazin],
1-[3-(2-Methoxyphenoxy)-2-hydroxypropyl]-spiro[piperidin-4,4'-2'-oxo-3',1'-benzooxazin],
1-[2-Phenyl-2-oxoethyl]-spiro[piperidin-4,4'-2'-oxo-3',1'-benzooxazin],
1-[4-(4-Fluorphenyl)-4-oxo-n-butyl]-spiro[piperidin-4,4'-2'-oxo-3',1'-benzooxazin],
1-[2-Phenyl-2-hydroxyethyl]-spiro[piperidin-4,4'-2'-oxo-3',1'-benzooxazin],
1-[3-(3-Methansulfamidophenyl)-2-hydroxyethyl]-spiro[piperidin-4,4'-2'-oxo-3',1'-benzooxazin],
1-[2-(4-Hydroxy-3-methoxycarbonylphenyl)-2-hydroxyethyl]-spiro[piperidin-4,4'-2'-oxo-3',1'-benzooxazin],
1-[2,6-Dichlorbenzyl]-spiro[piperidin-4,4'-2'-oxo-3',1'-benzooxazin],
1-(2-Phenylethyl)-spiro[piperidin-4,4'-2'-oxo-3',1'-benzooxazin],
oder einem pharmazeutisch annehmbares Säuresalz derselben hergestellt wird.

12. Verfahren zur Herstellung einer pharmazeutischen Zusammensetzung, dadurch gekennzeichnet, daß man eine Verbindung der Formel (I) gemäß Definition in einem der vorhergehenden Ansprüche oder ein pharmazeutisch annehmbares Säureadditionssalz derselben oder ein Produkt eines Verfahrens nach einem der vorhergehenden Ansprüche mit einem pharmazeutisch annehmbaren Träger oder Streckmittel mischt.

**Revendications pour les Etats contractants: BE CH DE FR GB IT LI LU NL SE**

1. Composé de formule

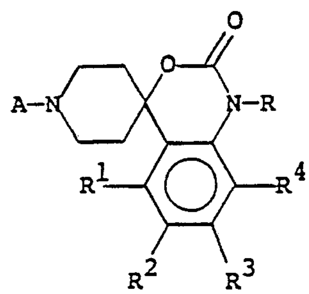

(I)

et les sels d'addition d'acides pharmaceutiquement acceptables de ce composé, formule dans laquelle

$R^1$, $R^2$, $R^3$ et $R^4$ représentent chacun, indépendamment, l'hydrogène, un radical hydroxy, alkyle en $C_1$ à $C_4$, alkoxy en $C_1$ à $C_4$ ou halogéno;

R est l'hydrogène ou un radical alkyle en $C_1$ à $C_4$; et

A est choisi entre:

un groupe 2-(benzodioxanne-2-yl)-2-hydroxyéthyle;

un groupe ω-(benzodioxanne-2-yl)-alkyle (où alkyle est un hydrocarbure à chaîne droite ou ramifié ayant 1 à 4 atomes de carbone);

un groupe 3-(aryloxy)-2-hydroxypropyle dont le radical aryloxy est un radical phényloxy éventuellement substitué par 1, 2 ou 3 radicaux choisis entre des radicaux alkyle en $C_1$ à $C_4$, alkoxy en $C_1$ à $C_4$, halogéno, (alkyle en $C_1$ à $C_4$)-sulfamido, (alkoxy en $C_1$ à $C_4$)-carbonyle, cyano et trifluorométhyle;

un groupe ω-arylalkyle dont le radical alkyle a la définition donnée ci-dessus et le radical aryle est est un radical phényle portant éventuellement 1, 2 ou 3 substituants choisis entre des radicaux alkyle en $C_1$ à $C_4$, alkoxy en $C_1$ à $C_4$, halogéno, (alkyle en $C_1$ à $C_4$)-sulfamido, (alkoxy en $C_1$ à $C_4$)-carbonyle, cyano et trifluorométhyle;

un groupe ω-aryl-ω-oxo-(alkyle en $C_1$ à $C_4$) dont les radicaux aryle et alkyle ont les définitions données ci-dessus;

un groupe ω-aryl-ω-hydroxy-(alkyle en $C_1$ à $C_4$) dans lequel les radicaux aryle et alkyle ont les définitions données ci-dessus; et

un groupe ω-aryloxyalkyle dont les radicaux alkyle et aryloxy ont les définitions données ci-dessus.

2. Composé suivant la revendication 1 ou un sel d'addition d'acide pharmaceutiquement acceptable de ce composé, dans lequel A est un groupe 2-(benzodioxanne-2-yl)-2-hydroxyéthyle ou ω-(benzodioxanne-2-yl)alkyle.

3. Composé suivant la revendication 1 ou un sel d'addition d'acide pharmaceutiquement acceptable de ce composé, dans lequel A est un groupe 3-(aryloxy)-2-hydroxypropyle ou ω-aryloxyalkyle.

4. Composé suivant la revendication 1 ou un sel d'addition d'acide pharmaceutiquement acceptable de ce composé, dans lequel A est un groupe ω-aryl-ω-oxoalkyle; ω-aryl-ω-hydroxyalkyle ou ω-aryloxyalkyle.

5. Composé suivant la revendication 1 ou un sel d'addition d'acide pharmaceutiquement acceptable de ce composé, dans lequel $R^1$, $R^2$, $R^3$ et $R^4$ représentent chacun indépendamment l'hydrogène ou un radical halogéno.

6. Composé suivant la revendication 1 ou un sel d'addition d'acide pharmaceutiquement acceptable de ce composé, dans lequel R est l'hydrogène ou un radical méthyle.

7. Composé suivant la revendication 2, la revendication 3 ou la revendication 4 ou un sel d'addition d'acide pharmaceutiquement acceptable de ce composé, dans lequel $R^1$, $R^2$, $R^3$ et $R^4$ représentent chacun indépendamment l'hydrogène ou un radical hydroxy, méthoxy ou halogéno; et R est l'hydrogène ou le radical méthyle.

8. La 1 - [2 - (benzodioxanne - 2 - yl) - 2 - hydroxyéthyl] - spiro - [pipéridine - 4,4' - 2' - oxo - 3',1' - benzooxazine)] ou un sel d'addition d'acide pharmaceutiquement acceptable de ce composé.

9. Composé suivant la revendication 7, choisi entre:

la 1-[2-(benzodioxanne-2-yl)-2-hydroxyéthyl]-1'-méthyl-spiro[pipéridine-4,4'-2'-oxo-3',1'-benzooxazine)],

la 1-[2-benzodioxanne-2-yl)-2-hydroxyéthyl]-6'-chloro-spiro[pipéridine-4,4'-2'-oxo-3',1'-benzooxazine)],

la 1-[2-(benzodioxanne-2-yl)-2-hydroxyéthyl]-6'-fluoro-spiro[pipéridine-4,4'-2'-oxo-3',1'-benzooxazine)],

la 1-[benzodioxanne-2-ylméthyl]-spiro[pipéridine-4,4'-2'-oxo-3',1'-benzooxazine],

la 1-[2-(benzodioxanne-2-yl)éthyl]-spiro[pipéridine-4,4'-2'-oxo-3',1'-benzooxazine],

la 1-[2-(benzodioxanne-2-yl)-2-hydroxyéthyl]-5'-hydroxy-spiro[pipéridine-4,4'-2'-oxo-3',1'-benzooxazine],

la 1-[2-(benzodioxanne-2-yl)-2-hydroxyéthyl]-5'-méthoxy-spiro[pipéridine-4,4'-2'-oxo-3',1'-benzooxazine],

la 1-[2-(2,4-diméthoxyphénoxy)éthyl]-spiro[pipéridine-4,4'-2'-oxo-3',1'-benzooxazine],

la 1-[2-phénoxyéthyl]-spiro[pipéridine-4,4'-2'-oxo-3',1'-benzooxazine],

la 1-[3-(2-cyanophénoxy)-2-hydroxypropyl]-spiro[pipéridine-4,4'-2'-oxo-3',1'-benzooxazine],

la 1-[3-phénoxy-2-hydroxypropyl]-spiro[pipéridine-4,4'-2'-oxo-3',1'-benzooxazine],

la 7'-fluoro-1-[3-(2-méthoxyphénoxy)-2-hydroxypropyl]-spiro[pipéridine-4,4'-2'-oxo-3',1'-benzooxazine],

la 1-[3-(2-méthoxyphénoxy)-2-hydroxypropyl]-spiro[pipéridine-4,4'-2'-oxo-3',1'-benzooxazine],

la 1-[2-phényl-2-oxoéthyl]-spiro[pipéridine-4,4'-2'-oxo-3',1'-benzooxazine],

la 1-[4-(4-fluorophényl)-4-oxo-n-butyl]-spiro[pipéridine-4,4'-2'-oxo-3',1'-benzooxazine],

la 1-[2-phényl-2-hydroxyéthyl]-spiro[pipéridine-4,4'-2'-oxo-3',1'-benzooxazine],

la 1-[3-(3-méthanesulfamidophényl)-2-hydroxyéthyl]-spiro[pipéridine-4,4'-2'-oxo-3',1'-benzooxazine],

la 1-[2-(4-hydroxy-3-méthoxycarbonylphényl)-2-hydroxyéthyl]-spiro[pipéridine-4,4'-2'-oxo-3',1'-benzooxazine],

la 1-[2,6-dichlorobenzyl]-spiro[pipéridine-4,4'-2'-oxo-3',1'-benzooxazine],

la 1-(phényléthyl)-spiro[pipéridine-4,4'-2'-oxo-3',1'-benzooxazine];

ou un sel d'addition d'acide pharmaceutiquement acceptable de ce composé.

10. Composition pharmaceutique, qui comprend une quantité thérapeutiquement efficace d'un composé suivant l'une quelconque des revendications précédentes ou d'un sel d'addition d'acide pharmaceutiquement acceptable de ce composé.

11. Composé suivant l'une quelconque des revendications 1 à 8 ou son sel d'addition d'acide pharmaceutiquement acceptable, destiné à être utilisé pour abaisser la pression sanguine chez des êtres humains.

12. Procédé de préparation d'un composé suivant la revendication 1, procédé qui consiste:

(a) à convertir un composé de formule

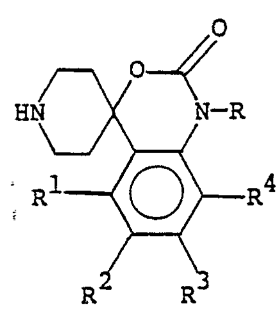

(IV)

dans laquelle $R^1$, $R^2$, $R^3$ et $R^4$ ont les définitions données ci-dessus et R est l'hydrogène, en un composé de formule IV dans laquelle R est un groupe alkyle en $C_1$ à $C_4$.

(b) à traiter un composé de formule IV dans laquelle $R^1$, $R^2$, $R^3$ et $R^4$ ont les définitions données ci-dessus et R est l'hydrogène ou un groupe alkyle en $C_1$ à $C_4$ avec un composé choisi entre

A'X et 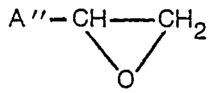

où A' est un groupe ω-(benzodioxanne-2-yl)alkyle, ω-aryloxyalkyle, ω-arylalkyle ou ω-aryl-ω-oxoalkyle; et A'' est un groupe benzodioxanne-2-yle ou un groupe aryloxyméthyle dont les radicaux alkyle et aryle sont tels que définis ci-dessus pour former un composé de formule I; ou

(c) à convertir un composé de formule I dans laquelle R est l'hydrogène et $R^1$, $R^2$, $R^3$ et $R^4$ ont les définitions données ci-dessus, en le produit correspondant de formule I dans laquelle R est un groupe alkyle en $C_1$ à $C_4$; ou

(d) à convertir un composé de formule I dans laquelle $R^1$, $R^2$, $R^3$ et $R^4$ ont les définitions données ci-dessus, R est l'hydrogène ou un groupe alkyle en $C_1$ à $C_4$ et A est un groupe ω-aryl-ω-oxyalkyle en le composé correspondant de formule I dans laquelle A est un groupe ω-aryl-ω-hydroxyalkyle; ou

(e) à convertir la base libre du composé de formule I en un sel d'addition d'acide pharmaceutiquement acceptable; ou

(f) à convertir un sel du composé de formule I en une base libre; ou

(g) à convertir un sel du composé de formule I en un autre sel.

13. Procédé de préparation d'un composé de formule:

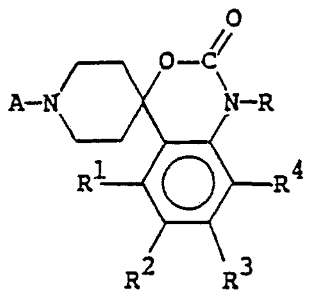

(I)

et de ses sels d'addition d'acides pharmaceutiquement acceptables, formule dans laquelle

$R^1$, $R^2$, $R^3$ et $R^4$ représentent chacun indépendamment l'hydrogène ou un radical hydroxy, alkyle en $C_1$ à $C_4$, alkoxy en $C_1$ à $C_4$ ou halogéno;

R est l'hydrogène ou un radical alkyle en $C_1$ à $C_4$; et

A est un groupe 2-(benzodioxanne-2-yl)-2-hydroxyéthyle;

procédé qui consiste:

(a) à traiter un composé de formule

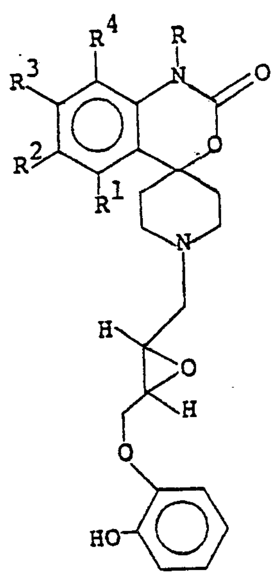

dans laquelle $R^1$, $R^2$, $R^3$, $R^4$ et R ont les définitions données ci-dessus, avec une base forte pour former un composé de formule I; ou

(b) à convertir un composé de formule I dans laquelle R est l'hydrogène en un composé de formule I dans laquelle R est un groupe alkyle en $C_1$ à $C_4$; ou

(c) a convertir la base libre du composé de formule I en un sel d'addition d'acide pharmaceutiquement acceptable; ou

(d) à convertir un sel du composé de formule I en une base libre; ou

(e) à convertir un sel du composé de formule I en un autre sel.

14. Procédé de préparation d'un composé de formule:

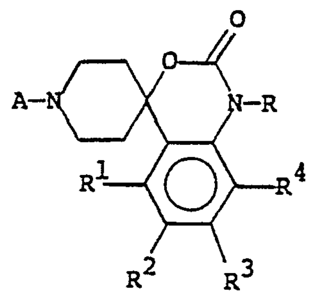

(I)

et de ses sels d'addition d'acides pharmaceutiquement acceptables, formule dans laquelle

$R^1$, $R^2$, $R^3$ et $R^4$ représentent chacun indépendamment l'hydrogène ou un radical hydroxy, alkyle en $C_1$ à $C_4$, alkoxy en $C_1$ à $C_4$ ou halogéno;

R est l'hydrogène; et

A est un groupe 2-(benzodioxanne-2-yl)-2-hydroxyéthyle;

procédé qui consiste:

a) à traiter un composé de formule

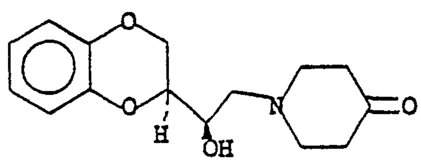

(XIV)

avec un composé de formule

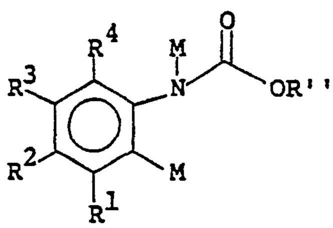

où $R^1$, $R^2$, $R^3$ et $R^4$ sont tels que définis ci-dessus, M est un métal, pour former un composé de formule I dans laquelle R est l'hydrogène et $R^1$, $R^2$, $R^3$ et $R^4$ ont les définitions données ci-dessus; puis

b) à éliminer la protection d'un composé de formule XVII

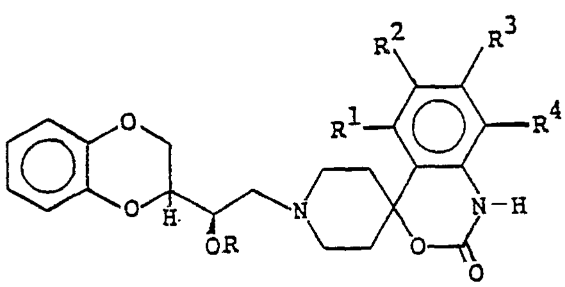

dans laquelle R est un groupe protégeant la fonction alcool et $R^1$, $R^2$, $R^3$ et $R^4$ ont les défintions données ci-dessus, pour obtenir un composé correspondant de formule I dans laquelle R est l'hydrogène, ou

c) à convertir un composé de formule XI

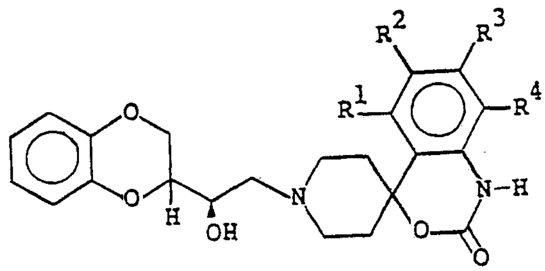

dans laquelle $R^1$, $R^2$, $R^3$ et $R^4$ ont les definitions données ci-dessus, en un composé correspondant de formule I dans laquelle R est un groupe alkyle en $C_1$ à $C_4$; ou bien

d) à convertir la base libre du composé de formule I en un sel d'addition d'acide pharmaceutiquement acceptable; ou

e) à convertir un sel du composé de formule I en une base libre; ou

f) à convertir un sel du composé de formule I en un autre sel.

15. Procédé de préparation d'une composition pharmaceutique, qui consiste à mélanger un produit d'un procédé suivant la revendication 12, 13 ou 14 avec un support ou excipient pharmaceutiquement acceptable.

**Revendications pour l'Etat contractant: AT**

1. Procédé de préparation d'un composé de formule

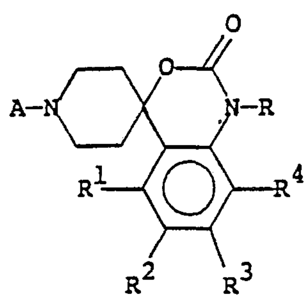

(I)

et de ses sels d'addition d'acides pharmaceutiquement acceptables, formule dans laquelle

$R^1$, $R^2$, $R^3$ et $R^4$ représentent chacun, indépendamment, l'hydrogène, un radical hydroxy, alkyle en $C_1$ à $C_4$, alkoxy en $C_1$ à $C_4$ ou halogéno;

R est l'hydrogène ou un radical alkyle en $C_1$ à $C_4$; et

A est choisi entre:

un groupe 2-(benzodioxanne-2-yl)-2-hydroxyéthyle;

un groupe ω-(benzodioxanne-2-yl)-alkyle (où alkyle est un hydrocarbure à chaîne droite ou ramifié ayant 1 à 4 atomes de carbone);

un groupe 3-(aryloxy)-2-hydroxypropyle dont le radical aryloxy est un radical phényloxy éventuellement substitué par 1, 2 ou 3 radicaux choisis entre des radicaux alkyle en $C_1$ à $C_4$, alkoxy en $C_1$ à $C_4$, halogéno, (alkyle en $C_1$ à $C_4$)-sulfamido, (alkoxy en $C_1$ à $C_4$)-carbonyle, cyano et trifluorométhyle;

un groupe ω-arylalkyle dont le radical alkyle a la définition donnée ci-dessus et le radical aryle est est un radical phényle portant éventuellement 1, 2 ou 3 substituants choisis entre des radicaux alkyle en $C_1$ à $C_4$, alkoxy en $C_1$ à $C_4$, halogéno, (alkyle en $C_1$ à $C_4$)-sulfamido, (alkoxy en $C_1$ à $C_4$)-carbonyle, cyano et trifluorométhyle;

un groupe ω-aryl-ω-oxoalkyle dont les radicaux alkyle et aryle ont les définitions données ci-dessus;

un groupe ω-aryl-ω-hydroxyalkyle dans lequel les radicaux alkyle et aryle ont les définitions données ci-dessus; et

un groupe ω-aryloxyalkyle dont les radicaux alkyle et aryloxy ont les définitions données ci-dessus.

procédé qui consiste:

a) à convertir un composé de formule

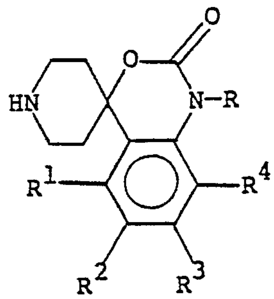

(IV)

dans laquelle $R^1$, $R^2$, $R^3$ et $R^4$ ont les définitions données ci-dessus et R est l'hydrogène, en un composé de formule IV dans laquelle R est un groupe alkyle en $C_1$à $C_4$.

(b) à traiter un composé de formule IV dans laquelle $R^1$, $R^2$, $R^3$ et $R^4$ ont les définitions données ci-dessus et R est l'hydrogène ou un groupe alkyle en $C_1$ à $C_4$ avec un composé choisi entre

A'X et 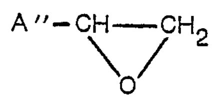

où A' est un groupe ω-(benzodioxanne-2-yl)alkyle, ω-aryloxyalkyle, ω-arylalkyle ou ω-aryl-ω-oxoalkyle; et A'' est un groupe benzodioxanne-2-yle ou un groupe aryloxyméthyle dont les radicaux alkyle et aryle sont tels que définis ci-dessus pour former un composé de formule I; ou

(c) à convertir un composé de formule I dans laquelle R est l'hydrogène et $R^1$, $R^2$, $R^3$ et $R^4$ ont les définitions données ci-dessus, en le produit correspondant de formule I dans laquelle R est un groupe alkyle en $C_1$ à $C_4$; ou

(d) à convertir un composé de formule I dans laquelle $R^1$, $R^2$, $R^3$ et $R^4$ ont les définitions données ci-dessus, R est l'hydrogène ou un groupe alkyle en $C_1$ à $C_4$ et A est un groupe ω-aryl-ω-oxyalkyle en le composé correspondant de formule I dans laquelle A est un groupe ω-aryl-ω-hydroxyalkyle; ou

(e) à convertir la base libre du composé de formule I en un sel d'addition d'acide pharmaceutiquement acceptable; ou

(f) à convertir un sel du composé de formule I en une base libre; ou

(g) à convertir un sel du composé de formule I en un autre sel.

2. Procédé de préparation d'un composé de formule:

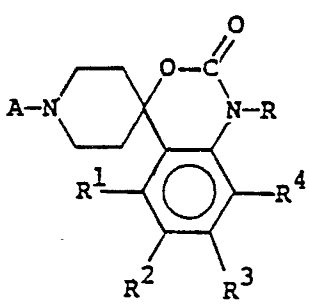

(I)

et de ses sels d'addition d'acides pharmaceutiquement acceptables, formule dans laquelle $R^1$, $R^2$, $R^3$ et $R^4$ représentent chacun indépendamment l'hydrogène ou un radical hydroxy, alkyle en $C_1$ à $C_4$, alkoxy en $C_1$ à $C_4$ ou halogéno;

R est l'hydrogène ou un radical alkyle en $C_1$ à $C_4$; et

A est un groupe 2-(benzodioxanne-2-yl)-2-hydroxyéthyle;

procédé qui consiste:

(a) à traiter un composé de formule

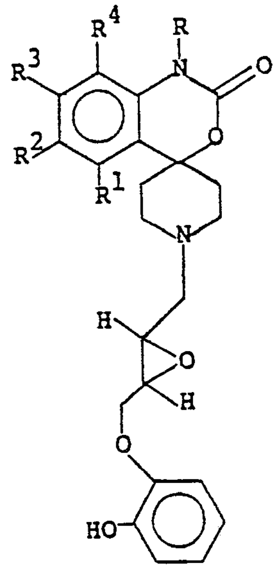

dans laquelle $R^1$, $R^2$, $R^3$, $R^4$ et R ont les définitions données ci-dessus, avec une base forte pour former un composé de formule I; ou

(b) à convertir un composé de formule I dans laquelle R est l'hydrogène en un composé de formule I dans laquelle R est un groupe alkyle en $C_1$ à $C_4$; ou

(c) a convertir la base libre du composé de formule I en un sel d'addition d'acide pharmaceutiquement acceptable; ou

(d) à convertir un sel du composé de formule I en une base libre; ou

(e) à convertir un sel du composé de formule I en un autre sel.

3. Procédé de préparation d'un composé de formule:

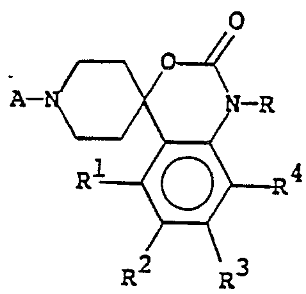

(I)

et de ses sels d'addition d'acides pharmaceutiquement acceptables, formule dans laquelle

$R^1$, $R^2$, $R^3$ et $R^4$ représentent chacun indépendamment l'hydrogène ou un radical hydroxy, alkyle en $C_1$ à $C_4$, alkoxy en $C_1$ à $C_4$ ou halogéno;

R est l'hydrogène; et

A est un groupe 2-(benzodioxanne-2-yl)-2-hydroxyéthyle;

procédé qui consiste:

a) à traiter un composé de formule

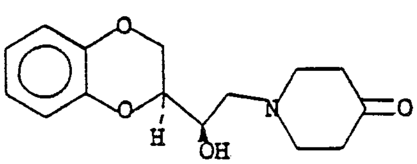

(XIV)

avec un composé de formule

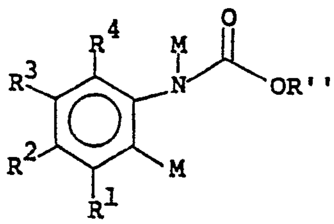

où $R^1$, $R^2$, $R^3$ et $R^4$ sont tels que définis ci-dessus, M est un métal, pour former un composé de formule I dans laquelle R est l'hydrogène et $R^1$, $R^2$, $R^3$ et $R^4$ ont les définitions données ci-dessus; puis

b) à éliminer la protection d'un composé de formule XVII

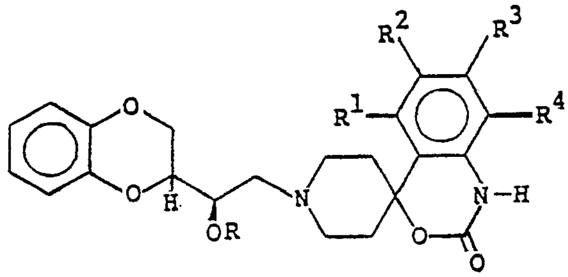

dans laquelle R est un groupe protégeant la fonction alcool et $R^1$, $R^2$, $R^3$ et $R^4$ ont les défintions données ci-dessus, pour obtenir un composé correspondant de formule I dans laquelle R est l'hydrogène, ou

c) à convertir un composé de formule XI

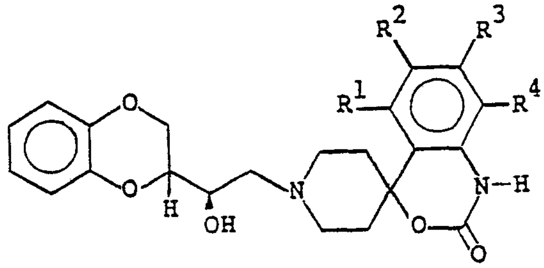

dans laquelle $R^1$, $R^2$, $R^3$ et $R^4$ ont les definitions données ci-dessus, en un composé correspondant de formule I dans laquelle R est un groupe alkyle en $C_1$ à $C_4$; ou bien

d) à convertir la base libre du composé de formule I en un sel d'addition d'acide pharmaceutiquement acceptable; ou

e) à convertir un sel du composé de formule I en une base libre; ou

f) à convertir un sel du composé de formule I en un autre sel.

4. Procédé suivant la revendication 1, dans lequel A est le groupe 2-(benzodioxanne-2-yl)-2-hydroxyéthyle ou un groupe ω-(benzodioxanne-2-yl)alkyle.

5. Procédé suivant la revendication 1, dans lequel A est un groupe 3-(aryloxy)-2-hydroxypropyle ou ω-aryloxyalkyle.

6. Procédé suivant la revendication 1, dans lequel A est un groupe ω-aryl-ω-oxoalkyle; ω-aryl-ω-hydroxyalkyle ou ω-aryloxyalkyle.

7. Procédé suivant la revendication 1, dans lequel $R^1$, $R^2$, $R^3$ et $R^4$ représentent chacun indépendamment l'hydrogène ou un radical halogéno.

8. Procédé suivant la revendication 1, dans lequel R est l'hydrogène ou le groupe méthyle.

9. Procédé suivant l'une quelconque des revendications 4, 5 et 6, dans lequel $R^1$, $R^2$, $R^3$, $R^4$ représentent chacun indépendamment l'hydrogène ou un radical hydroxy, méthoxy ou halogéno; et R est l'hydrogène ou le radical méthyle.

10. Procédé suivant la revendication 1, dans lequel la 1 - [2 - (benzodioxanne - 2 - yl) - 2 - hydroxyéthyl] - spiro]pipéridine - 4,4' - 2' - oxo - 3',1' - benzooxazine)] ou un sel d'addition d'acide pharmaceutiquement acceptable de ce composé est produit.

11. Procédé suivant l'une quelconque des revendications 1 à 9, dans lequel est produit un composé de formule (I) choisi entre

la 1-[2-(benzodioxanne-2-yl)-2-hydroxyéthyl]-1'-méthyl-spiro[pipéridine-4,4'-2'-oxo-3',1'-benzooxazine)],

la 1-[2-benzodioxanne-2-yl)-2-hydroxyéthyl]-6'-chloro-spiro[pipéridine-4,4'-2'-oxo-3',1'-benzooxazine)],

la 1-[2-(benzodioxanne-2-yl)-2-hydroxyéthyl]-6'-fluoro-spiro[pipéridine-4,4'-2'-oxo-3',1'-benzooxazine)],

la 1-[benzodioxanne-2-ylméthyl]-spiro[pipéridine-4,4'-2'-oxo-3',1'-benzooxazine],

la 1-[2-(benzodioxanne-2-yl)éthyl]-spiro[pipéridine-4,4'-2'-oxo-3',1'-benzooxazine],

la 1-[2-(benzodioxanne-2-yl)-2-hydroxyéthyl]-5'-hydroxy-spiro[pipéridine-4,4'-2'-oxo-3',1'-benzooxazine],

la 1-[2-(benzodioxanne-2-yl)-2-hydroxyéthyl]-5'-méthoxy-spiro[pipéridine-4,4'-2'-oxo-3',1'-benzooxazine],

la 1-[2-(2,4-diméthoxyphénoxy)éthyl]-spiro[pipéridine-4,4'-2'-oxo-3',1'-benzooxazine],

la 1-[2-phénoxyéthyl]-spiro[pipéridine-4,4'-2'-oxo-3',1'-benzooxazine],

la 1-[3-(2-cyanophénoxy)-2-hydroxypropyl]-spiro[pipéridine-4,4'-2'-oxo-3',1'-benzooxazine],

la 1-[3-phénoxy-2-hydroxypropyl]-spiro[pipéridine-4,4'-2'-oxo-3',1'-benzooxazine],

la 7'-fluoro-1-[3-(2-méthoxyphénoxy)-2-hydroxypropyl]-spiro[pipéridine-4,4'-2'-oxo-3',1'-benzooxazine],

la 1-[3-(2-méthoxyphénoxy)-2-hydroxypropyl]-spiro[pipéridine-4,4'-2'-oxo-3',1'-benzooxazine],

la 1-[2-phényl-2-oxoéthyl]-spiro[pipéridine-4,4'-2'-oxo-3',1'-benzooxazine],

la 1-[4-(4-fluorophényl)-4-oxo-n-butyl]-spiro[pipéridine-4,4'-2'-oxo-3',1'-benzooxazine],

la 1-[2-phényl-2-hydroxyéthyl]-spiro[pipéridine-4,4'-2'-oxo-3',1'-benzooxazine],

la 1-[3-(3-méthanesulfamidophényl)-2-hydroxyéthyl]-spiro[pipéridine-4,4'-2'-oxo-3',1'-benzooxazine],

la 1-[2-(4-hydroxy-3-méthoxycarbonylphényl)-2-hydroxyéthyl]-spiro[pipéridine-4,4'-2'-oxo-3',1'-benzooxazine],

la 1-[2,6-dichlorobenzyl]-spiro[pipéridine-4,4'-2'-oxo-3',1'-benzooxazine],

la 1-(2-phényléthyl)-spiro[pipéridine-4,4'-2'-oxo-3',1'-benzooxazine];

ou un sel d'addition d'acide pharmaceutiquement acceptable de ce composé.

12. Procédé de préparation d'une composition pharmaceutique, qui consiste à mélanger un composé de formule (I) tel que défini dans l'une quelconque des revendications précédentes ou un sel d'addition d'acide pharmaceutiquement acceptable de ce composé, ou un produit d'un procédé selon l'une quelconque des revendications précédentes avec un support ou excipient pharmaceutiquement acceptable.